(19) Europäisches Patentamt
European Patent Office
Office européen des brevets

(11) **EP 3 055 031 B1**

(12) **FASCICULE DE BREVET EUROPEEN**

(45) Date de publication et mention de la délivrance du brevet:
**13.03.2019 Bulletin 2019/11**

(21) Numéro de dépôt: **14790225.8**

(22) Date de dépôt: **07.10.2014**

(51) Int Cl.:
*A61Q 1/00* *(2006.01)*   *A61Q 5/00* *(2006.01)*
*A61Q 19/00* *(2006.01)*   *A61K 8/81* *(2006.01)*
*A61Q 19/10* *(2006.01)*   *A61Q 5/02* *(2006.01)*

(86) Numéro de dépôt international:
**PCT/FR2014/052536**

(87) Numéro de publication internationale:
**WO 2015/052426 (16.04.2015 Gazette 2015/15)**

(54) **UTILISATION EN COSMETIQUE DE POLYMERES OBTENUS PAR POLYMERISATION EN EMULSION INVERSE BASSE CONCENTRATION**

VERWENDUNG VON POLYMEREN HERGESTELLT DURCH INVERSE EMULSIONSPOLYMERISATION

USE OF POLYMERS OBTAINED BY LOW-CONCENTRATION INVERSE EMULSION POLYMERIZATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.10.2013 FR 1359693**

(43) Date de publication de la demande:
**17.08.2016 Bulletin 2016/33**

(73) Titulaire: **S.P.C.M. SA**
**42160 Andrézieux Bouthéon (FR)**

(72) Inventeurs:
• **BLONDEL, Frédéric**
  **F-42600 Lezigneux (FR)**
• **CHAMPAGNON, Lionel**
  **F-42600 Magneux Haute Rive (FR)**

(74) Mandataire: **Denjean, Eric**
**Cabinet Laurent et Charras**
**Le Contemporain**
**50, chemin de la Bruyère**
**69574 Dardilly (FR)**

(56) Documents cités:
**US-A- 4 539 368**   **US-A- 4 656 222**
**US-A- 5 216 070**   **US-A- 5 380 465**
**US-A1- 2003 147 825**

Remarques:
Le dossier contient des informations techniques présentées postérieurement au dépôt de la demande et ne figurant pas dans le présent fascicule.

**Description**

L'invention concerne le domaine technique des compositions cosmétiques ou dermatologiques et a, plus précisément, pour objet l'utilisation dans ce domaine de polymères acryliques synthétiques comprenant au moins une fonction acide faible, obtenus dans des conditions particulières par le procédé de polymérisation en émulsion inverse à partir d'au moins un monomère porteur d'une fonction acide faible, ainsi que les compositions cosmétiques correspondantes.

Les compositions cosmétiques ou dermatologiques comportent en général une phase aqueuse. Elles sont appliquées notamment sur la peau ou les cheveux et se présentent généralement sous la forme d'émulsions huile dans eau, et parfois eau dans huile, pour former par exemple des crèmes ou des lotions. De telles compositions peuvent, par exemple, correspondre à des crèmes ou lotions anti-âge, des soins après-rasage, des crèmes hydratantes, des lotions colorantes pour cheveux, des shampoings, des après-shampoings, des shampoings conditionneurs, des produits de douche, des crèmes nettoyantes, ou encore des pommades ou des crèmes ou lotions solaires. Les crèmes se distinguent des lotions par leur plus forte viscosité.

Les épaississants ou modificateur de rhéologie sont largement utilisés dans ces compositions pour adapter leur profil sensoriel (aspect, application) aux exigences du consommateur, mais aussi pour suspendre ou stabiliser des principes actifs.

Pour cela, différents types d'agent épaississant/viscosant ont déjà été proposés :

- Des gommes naturelles et/ou modifiées, comme la guar, i'hydroxyéthyl cellulose, ou encore des biopolymères tel que le xanthane, sont utilisés. Ces polymères, qui sont non ioniques à légèrement anioniques, sont peu sensibles aux espèces chargées souvent présentes dans les compositions (EDTA, principe actif ionique...), mais sont intrinsèquement peu efficaces et connus comme procurant des textures peu attrayantes et comme ayant tendance à procurer, durant l'application, une sensation de collant /gluant (« tackiness » en anglais) et à laisser un film après séchage.

- Des produits synthétiques de type Carbomer, homopolymère d'acide acrylique réticulé obtenus par polymérisation par précipitation sont également utilisés, Néanmoins, leur méthode d'obtention induit la présence de solvants organiques résiduels de type benzène, solvants chlorés ou encore cyclohexane qui ne présentent pas des profils toxicologiques satisfaisants pour l'application cosmétique. De plus, il s'agit de polymères qui nécessitent une étape de neutralisation lors de la mise en oeuvre par le formulateur pour pouvoir développer des propriétés épaississantes. Ce type de polymère peut présenter un bon pouvoir suspensif mais reste inefficace pour l'émulsification de phase grasse. Le brevet US 5,004,598, au nom de The BF Goodrich Company, propose pour cela un copolymère sur la base d'acide acrylique avec un faible part de monomère hydrophobe, procurant ainsi des propriétés émulsifiantes, mais peu viscosantes.

- L'utilisation de poudres pré-neutralisées et des dispersions liquides dans des huiles organiques de (co)polymères acryliques, obtenues par polymérisation en émulsion inverse est également proposée, Ces polymères ont une bien meilleure aptitude à émulsifier des phases grasses, mais demeurent aussi moins efficaces qu'un Carbomer et entraînent des problèmes de résidus lors de l'application. La demande de brevet WO 2005/097834 au nom de CIBA propose d'améliorer les propriétés viscosantes (efficacité) en neutralisant les monomères porteurs d'une fonction acide avant polymérisation, avec un taux de neutralisation de 25 à 100%, et préférentiellement de 30 à 40%.

Les brevets EP 0 503 853 et EP 1 047 716 proposent des émulsions inverses de polymères à base d'ATBS (l'acide 2-acrylamido-2-méthylpropane sulfonique) permettant de viscosifier des compositions cosmétiques ou dermatologiques y compris à pH acide. Même si ces polymères, ainsi que ceux décrits dans la demande WO 2005/097834, ont permis d'améliorer l'efficacité épaississante dans ce type de composition, ils ne répondent pas encore aux exigences des utilisateurs. Ces utilisateurs sont à la recherche de nouvelles solutions plus efficaces, i.e. proposant une meilleure efficacité épaississante avec éventuellement un dosage moindre en polymère, tout en permettant une plus grande latitude de formulations avec des composés ioniques. De plus les polymères contenant une grande quantité d'ATBS sont chers, et un autre enjeu est de proposer des polymères à moindre coût.

Il a été constaté que la résistance aux électrolytes de la plupart de ces polymères est généralement faible, du fait de leur caractère fortement anionique. Ils présentent, de ce fait, une forte sensibilité aux électrolytes. Or, les compositions cosmétiques ou dermatologiques contiennent des électrolytes.

Les électrolytes sont des substances chimiques chargés positivement ou négativement et capable de transporter ou conduire une charge électrique, généralement dans une solution. On les appelle également des composés ioniques et dans le domaine de la formulation de compositions cosmétiques et dermatologiques, des ingrédients ioniques. Ils peuvent être monovalents ou multivalents. Les électrolytes sont principalement des acides, des bases ou des sels. Plus précisément parmi les électrolytes souvent rencontrés dans les compositions cosmétiques et dermatologiques, on peut citer des adjuvants comme les extraits végétaux contenant des ions monovalent ou divalents, les principes actifs tels que les hydroxy acides pour leur effet anti-âge, les agents hydratants tels que l'acide carboxylique pyrrolidone (PCA), des agents chelatants comme par exemple l'acide éthylène diamine tétraacétique (EDTA), les filtres UV comme l'acide sulfonique phenylbenzimidazole, certains conservateurs ou encore des sels comme par exemple le sel d'alun. Ces électrolytes présents dans les compositions affectent donc l'efficacité des épaississants se traduisant par une forte diminution de la viscosité de la composition. Il est alors nécessaire d'augmenter la dose de polymères épaississant pour obtenir un épaississement satisfaisant de la composition.

Par ailleurs, l'épiderme sur lequel sont appliquées les crèmes, shampoings ou lotions contient également des électrolytes comme par exemple le chlorure de sodium. L'application d'une crème ou d'une lotion ne résistant pas suffisamment aux électrolytes donnera alors une désagréable sensation de liquéfaction au contact de la peau.

Pour répondre à cette problématique, la demande de brevet US 2003/0147825 au nom de NOVEON propose d'utiliser un polymère sous forme d'émulsion inverse comprenant un monomère acrylique amphiphile. Cependant, ces polymères conduisent à des textures de crèmes plutôt structurées, voire gélifiées, ne correspondant pas aux attentes du consommateur. Il est à noter que les monomères contenant des groupements acide carboxylique sont neutralisés à hauteur de 60 à 100%, et de 75 à 95% avant la réaction de polymérisation (voir paragraphe [0042]), ce qui correspond aux conditions classiquement mises en oeuvre. En effet, il est mentionné dans différents documents traitant de la préparation de polymères acryliques directement réalisée par un procédé de polymérisation en émulsion inverse eau dans huile (comme notamment explicité dans le document US 5,216,070), que, pour des raisons de faisabilité, il est nécessaire dans les cas où les polymères souhaités sont réalisés à partir d'au moins un monomère comportant une fonction acide faible tel que l'acide acrylique, d'utiliser pour réaliser la réaction de polymérisation, des monomères dont la fonction acide faible est sous forme neutralisée, afin d'éviter les problèmes de précipitation lors de l'utilisation d'un procédé de préparation par émulsion inverse. Les brevets US 5,380,465, US 4,539,368 et US 4,656,222, et la publication de Chinese Chemical Letters Vol. 13, N°10, pp 993-996, 2002, par exemple, utilisent tous un pourcentage de neutralisation important, voire total, des monomères porteurs d'une fonction acide faible pour réaliser une réaction de polymérisation en émulsion inverse. En effet, comme indiqué dans notamment dans le brevet US 5,216,070, la préparation de tels polymères en l'absence de neutralisation des monomères porteurs d'une fonction acide faible utilisés, directement par le procédé de polymérisation en émulsion inverse, pose un problème de précipitation/déstabilisation.

Il apparait qu'il existe donc un réel besoin pour améliorer les compositions cosmétiques et dermatologiques existantes. Le but étant de proposer des compositions présentant un aspect attrayant, une stabilité satisfaisante, qui soit adaptée à l'incorporation de tout type d'ingrédients ioniques (mono ou multivalent), et donc présenter une excellente résistance aux électrolytes. Dans ce contexte, un des objectifs de la demanderesse a été de développer des polymères obtenus par polymérisation en émulsion inverse présentant des performances épaississantes améliorées, mais surtout une meilleure résistance aux électrolytes classiquement utilisés dans les compositions cosmétiques et dermatologiques. En d'autres termes, un des objectifs de la demanderesse est l'obtention d'un effet épaississant des compositions cosmétiques ou dermatologiques en présence d'électrolytes tout en diminuant la quantité de polymère nécessaire et en gardant un aspect attrayant des compositions.

L'objet de cette invention est donc de proposer pour épaissir des compositions cosmétiques ou dermatologiques, l'utilisation d'un polymère acrylique obtenu par mises en oeuvre de conditions particulières dans un procédé de polymérisation en émulsion inverse, ledit polymère ayant une résistance aux électrolytes améliorée, tout en conservant une bonne efficacité épaississante et en donnant un aspect attrayant aux compositions.

La présente invention concerne l'utilisation, pour la fabrication d'une composition cosmétique ou dermatologique comprenant au moins une phase aqueuse, d'un polymère branché ou réticulé composé de la répétition d'une ou plusieurs unités monomériques, avec au moins une des unités monomériques qui correspond à un monomère comportant un groupe acrylique et au moins 30% molaire des unités monomériques qui sont porteuses d'au moins une fonction acide faible éventuellement sous forme neutralisée, ledit polymère étant obtenu :

par polymérisation d'une solution aqueuse d'un ou plusieurs monomères en émulsion inverse eau dans huile, au moins un des monomères utilisés étant un monomère acrylique et un ou plusieurs des monomères utilisés étant un monomère porteur d'au moins une fonction acide faible, le pourcentage molaire de monomères porteurs d'au moins une fonction acide faible par rapport à l'ensemble des monomères utilisés étant au moins de 38%, la phase aqueuse contenant au moins un monomère jouant le rôle d'agent de ramification, de sorte que la polymérisation conduit à un polymère branché ou réticulé, caractérisé en ce que :

i) la polymérisation est réalisée avec une concentration de la totalité des monomères en solution aqueuse appartenant à la gamme allant de 1,3 mmol à 3,6 mmol par gramme de solution aqueuse,
ii) pendant la polymérisation, au plus 20% des fonctions acide présentes sur les monomères possédant au moins une fonction acide se trouvent sous forme neutralisée ;

la polymérisation étant éventuellement suivie d'une ou plusieurs des étapes suivantes :

- une dilution ou une concentration de l'émission obtenue,
- une isolation pour obtenir le polymère sous la forme d'une poudre,
- une neutralisation au moins partielle des fonctions acide libre présentes dans le polymère obtenu,

Un tel polymère défini par son procédé d'obtention est nommé dans la suite de la description « polymère épaississant », « polymère acrylique » ou « polymère branché ou réticulé ».

L'invention a également pour objet l'utilisation d'un tel polymère épaississant, pour épaissir, voire épaissir et émulsionner, une composition cosmétique ou dermatologique comprenant au moins une phase aqueuse. De manière préférée, pour obtenir l'effet épaississant souhaité, le polymère épaississant comporte un pourcentage de fonctions acide neutralisées de 30 à 100% par rapport à la totalité des fonctions acide présentes sur le polymère, obtenu par une étape de neutralisation au moins partielle des fonctions acides présentes sur le polymère réalisée après la polymérisation, mais avant ou après la préparation de la composition.

L'invention concerne également les compositions cosmétiques ou dermatologiques comprenant au moins une phase aqueuse et un tel polymère épaississant, la polymérisation étant suivie d'une étape de neutralisation au moins partielle des fonctions acides présentes réalisée avant ou après l'incorporation du polymère dans la composition ; et éventuellement d'une ou plusieurs des étapes suivantes, réalisées avant l'incorporation du polymère dans la composition :

- une dilution ou une concentration de l'émulsion obtenue,
- une isolation pour obtenir le polymère sous la forme d'une poudre,

L'utilisation d'une composition selon l'invention pour le traitement cosmétique ou dermatologique des matières kératiniques telles que la peau, le cuir chevelu, les cils, les sourcils, les ongles, les cheveux et/ou les muqueuses, à l'exclusion de tout traitement thérapeutique, fait également partie intégrante de l'invention. Une telle utilisation comprend l'application de la composition sur les matières kératiniques, éventuellement suivie d'un rinçage à l'eau.

Les utilisations et compositions selon l'invention présentent, de préférence, l'une ou l'autre des caractéristiques ci-après, ou une combinaison quelconque de ces caractéristiques, voire toutes les caractéristiques ci-dessous lorsqu'elles ne s'excluent pas l'une l'autre :

- pendant la polymérisation, au plus 10%, de préférence au plus 5%, et préférentiellement au plus 2%, des fonctions acide présentes sur les monomères possédant au moins une fonction acide se trouvent sous forme neutralisée ; Selon, un mode de réalisation particulier, toutes les fonctions acide présentes sur les monomères se trouvent sous forme acide libre, pendant la polymérisation ;
- la polymérisation est réalisée avec une concentration de la totalité des monomères en solution aqueuse appartenant à la gamme allant de 1,7 à 3,3 mmol par gramme de solution aqueuse ;
- le polymère comporte un pourcentage molaire d'unités monomériques porteuses d'une ou plusieurs fonction(s) acide faible, par rapport à l'ensemble des unités monomériques porteuses d'une fonction acide, d'au moins 50 %, préférentiellement d'au moins 70%, très préférentiellement d'au moins 80% ;
- tous les monomères utilisés pour la préparation du polymère sont des monomères possédant au moins une insaturation éthylénique ;
- la ou les unité(s) monoménque(s) porteuse(s) d'au moins une fonction acide faible, sous forme libre, est(sont) choisie(s) parmi l'acide acrylique, l'acide méthacrylique, l'acide itaconique, l'acide crotonique, l'acide maléique et l'acide fumarique, l'acide acrylique étant préféré ;
- le polymère est un copolymère comportant au moins une unité monomérique neutre choisie parmi l'acrylamide, le méthacrylamide, le N,N-diméthylacrylamide, le N-vinylméthylacétamide, le N-vinylformamide, l'acétate de vinyle, le diacétoneacrylamide, le N-isopropyl acrylamide, le N-[2-hydroxy-1,1~bis(hydroxyméthyl)éthyl] propénami0e, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle) et la vinylpyrrolidone ;
- soit toutes les unités monomériques porteuses d'au moins une fonction acide présentes dans le polymère sont des unités monomériques porteuses d'une ou plusieurs fonction(s) acide faible. En particulier, le polymère présent dans la composition est un copolymère acide acrylique/acrylamide avec de 30 à 100% des fonctions acide acrylique sous forme neutralisée ; soit le polymère est un copolymère comportant au moins une unité monomérique porteuse d'une ou plusieurs fonctlon(s) acide fort. De manière préférée, le pourcentage molaire en unités monomériques porteuses d'une ou plusieurs fonction(s) acide fort par rapport à l'ensemble des unités monomériques est inférieur à 50%, et préférentiellement inférieur à 30% Par exemple, la ou les unité(s) monomérique(s) porteuse(s) d'une ou plusieurs fonction(s) acide fort, sous forme libre, est(sont) choisie(s) parmi les acides acrylamidoalkylsulfonique tel que l'acide 2-acrylamido-2-méthylpropane sulfonique. En particulier, le polymère présent dans la composition est un copolymère acide 2-acrylamido-2-méthylpropane sulfonique/acide acrylique ou acide 2-acrylamido-2-méthylpropane sulfonique/acide acrylique/acrylamide, avec de 30 à 100% des fonctions acide présentes sur le polymère qui sont sous forme neutralisée ;

- l'agent de ramification est choisi parmi le méthylènebisacrylamide (MBA), l'éthylène glycol di-acrylate, le polyéthylène glycol diméthacrylate, le diacryamide, le cyanométhylacrylate, le vinyloxyéthylacrylate, le vinyloxyméthacrylate, la triallylamine, le formaldéhyde, le glyoxal, les glycidyléthers comme l'éthylèneglycol diglycidyléther, les époxy et leur mélange ;
- la quantité d'agent de ramification est comprise entre 5 et 10 000 ppm en masse par rapport à la masse totale de monomères, et préférentiellement entre 100 et 5 000 ppm ;
- la réaction de polymérisation est menée en présence d'un agent émulsionnant eau dans huile ;
- la polymérisation est menée avec un agent de transfert, par exemple, choisi parmi le méthanol, l'alcool isopropylique, l'hypophosphite de sodium, le 2-mercaptoéthanol, le méthallysulfonate de sodium et leur mélange ; De préférence, la quantité d'agent de transfert est comprise entre 0 et 5000 ppm en masse par rapport à la masse totale de monomères, et préférentiellement entre 10 et 2500 ppm ;
- le polymère branché ou réticulé utilisé dans la composition présente une viscosité, à 0,16% en masse dans de l'eau déminéralisée dont le pH est ajusté à 7 +/-0,1 avec de l'hydroxyde de sodium, mesurée à 25°C avec un appareil Brookfiled de type RVT (vitesse de rotation 20 t/min), appartenant à la gamme allant de 2000 mPa.s à 100 000 mPa.s, notamment à la gamme allant de 3000 mPa.s à 50 000 mPa.s. La procédure de mesure de la viscosité de la solution aqueuse de polymère à 0,16 % en masse est la suivante. Dans un bécher de 400 ml, on introduit 250 g d'eau déionisée, puis sous agitation mécanique (tripâle - 500 tours par minute), on ajoute progressivement sous agitation la quantité de polymère désirée pour obtenir une solution contenant 0,16% en masse de polymère actif. De préférence, le polymère ajouté se trouve dans la forme (émulsion inverse, poudre sèche, solution dans l'eau ...) sous laquelle il est utilisé pour la préparation de la composition cosmétique ou dermatologique. Le pH est ensuite ajusté à 7 +/-0,1 avec de l'hydroxyde de sodium. A ce pH, 100% des fonctions acide présentes sur le polymère sont neutralisées. La solution est laissée 15 minutes sous agitation puis 5 minutes au repos. La viscosité est alors mesurée à l'aide d'un viscosimètre Brookfield de type RVT (vitesse de rotation 20 tours par minute). Le polymère branché ou réticulé présent dans la composition permet ainsi d'épaissir la composition à une certaine viscosité, mesurée à 25°C avec un appareil Brookfiled, appartenant à la gamme allant de 100 mPa.s à 100,000 mPa.s, notamment à la gamme allant de 100 mPa.s à 50.000 mPa.s. En particulier, les émulsions, lotions et crèmes selon l'invention auront une viscosité appartenant à la gamme allant de 1.000 mPa.s à 50.000 mPa.s, préférentiellement à la gamme allant de 5.000 mPa.s à 40.000 mPa.s. Les shampoings, après-shampoing et produits de douche selon l'invention auront une viscosité appartenant à la gamme allant de 100 mPa.s à 10.000 mPa.s, préférentiellement à la gamme allant de 300 mPa.s à 5.000 mPa.s ;
- la composition est adaptée pour une application topique. La composition se présente, par exemple, sous la forme d'un lait, d'une lotion, d'un gel, d'une crème, d'un gel crème, d'un savon, d'un bain moussant, d'un baume, d'un shampooing ou d'un après-shampooing, d'un produit de douche ;
- la composition comprend un électrolyte, de préférence, choisi parmi les extraits végétaux contenant des ions monovalents ou divalents comme les acides de fruits, les principes actifs tels que les hydroxy acides pour leur effet anti-âge, les agents hydratants tels que l'acide carboxylique pyrrolidone, des agents chelatants comme l'acide éthylène diamine tétraacétique (EDTA), les filtres UV comme l'acide sulfonique phénylbenzimidazole, certains conservateurs ou encore des sels comme le sel d'alun ;
- la composition comprend de 0,01% à 10% en masse de polymère branché ou réticulé, par rapport à la masse totale de la composition, et préférentiellement de 0,1 à 5% en masse de polymère branché ou réticulé ;
- la composition comprend au moins un agent actif choisi parmi choisi parmi les agents hydratants, les agents bronzants, les filtres solaires, les vitamines, les oligo-éléments, les agents anti-rides ou anti-âge, les extraits botaniques, les agents à visée amincissante, les agents anti-radicalaires, les agents anti-chutes des cheveux, les agents antipelliculaires, les polymères de conditionnement de la peau, les tensioactifs nettoyants, les émollients et les principes actifs pharmaceutiques tels que les agents anti-fongiques, les agents anti-bactériens, les agents anti-inflammatoires, les myorelaxants, les antibiotiques, les agents antiviraux, les analgésiques, les antihistaminiques, les agents antipruritiques, les agents antipyrétiques, les agents anesthésiques, les agents diagnostiques, les hormones, les rehausseurs de croissance cutanée, les modulateurs pigmentaires, les agents antiprolifératifs, les agents antipsoriatiques, les rétinoïdes, les médicaments anti-acné, les agents antinéopastiques, les agents photothérapeutiques, les agents kératolytiques, et leurs analogues ;
- la composition comprend au moins un additif, et notamment au moins un auxiliaire de formulation, par exemple choisi parmi les agents chélatants, les diluants, les agents de neutralisation et d'ajustement du PH, les opacifiants, les conservateurs, les agents d'étalement, les émollients, les polymères filmogènes, les antioxydants, les parfums, les agents réfléchissants, les agents coalescents et les mélanges de ceux-ci ;
- la composition est une émulsion d'une phase huileuse dans une phase aqueuse ou une émulsion d'une phase aqueuse dans une phase huileuse ;

- la phase huileuse est constituée d'une huile végétale ou de plante, d'une huile silicone, d'une huile hydrocarbonée fluorée, d'une huile hydrocarbonée, d'une huile minérale, de polyisobutène, d'isohexadécane, d'un triglycéride caprylique/caprique, d'octanoate de cétéaryle, de benzoate d'alkyle en $C_{12}$-$C_{14}$, ou d'un de leur mélange ;
- la composition comprend un agent émulsionnant eau dans huile et/ou un agent émulsionnant huile dans eau.

Les polymères épaississants utilisés dans le cadre de l'invention et leur procédé d'obtention vont tout d'abord être décrits.

Les polymères utilisés dans le cadre de l'invention, sont composés de la répétition d'une ou plusieurs unités monomériques, avec au moins une des unités monomériques qui correspond à un monomère comportant un groupe acrylique. En d'autres termes, ils correspondent à des homopolyrnères obtenus par polymérisation d'un monomère comportant un groupe acrylique ou à des copolymères obtenus par copolymérisation d'un mélange de monomères, dont un au moins comporte un groupe acrylique. Par souci de simplicité, dans la suite de la description, de tels polymères pourront simplement être nommés polymères acryliques.

De manière à remplir efficacement leur rôle d'épaississant, les polymères utilisés dans le cadre de l'invention sont hydrosolubles ou hydro-gonflants et vont donc se trouver dans la phase aqueuse de la composition. Les monomères utilisés pour la préparation de ces polymères et en particulier le taux de monomères hydrophiles seront sélectionnés, de manière à obtenir de telles propriétés.

Par polymère hydrosoluble, on entend un polymère qui, mis en solution à l'aide d'une agitation dans de l'eau à une température de 25°C à une concentration de 50g/l, donne une solution exempte de particules insolubles.

Par polymère hydro-gonflant, on entend un polymère qui, mis en solution dans de l'eau à une température de 25°C, gonfle et épaissit la solution.

Les polymères utilisés dans le cadre de l'invention sont branchés ou réticulés. Par polymères branchés, on entend, de manière classique, les polymères non linéaires qui possèdent des chaînes latérales, Les polymères branchés, incluent notamment les polymères en forme d'étoile (star) et en forme de peigne (comb). Par polymère réticulé, on entend, de manière classique, un polymère non linéaire qui se présente sous la forme d'un réseau tridimensionnel insoluble dans l'eau, mais gonflable dans l'eau.

La rétlculation est obtenue en mettant en oeuvre un agent de ramification lors de la polymérisation qui est intégré dans la phase aqueuse Un tel agent de ramification correspond à un monomère comportant deux ou plus d'insaturations éthyléniques et est, par exemple, choisi parmi le méthylènebisacrylamide (MBA), l'éthylène glycol di-acrylate, le polyéthylène glycol diméthacrylate, le diacrylamide, le cyanométhylacrylate, le vinyioxyéthylacrylate, le vinyloxyméthacrylate, la triallylamine, le formaldéhyde, le glyoxal, les glycidyléthers comme réthylèneglycol diglycidyiéther, les époxy et leur mélange.

Il convient de préciser que, dans le cadre de l'invention, la concentration totale en monomères donnée en relation avec le procédé de polymérisation Inclut les monomères jouant le rôle d'agent de ramification.

Dans le cadre de l'invention, la demanderesse s'est intéressée à l'utilisation de polymères acryliques correspondants ou obtenus à partir d'une émulsion inverse de polymères préparée par polymérisation en émulsion inverse eau dans huile avec utilisation d'un pourcentage molaire élevé de monomères porteurs d'une ou plusieurs fonction(s) acide faible par rapport à l'ensemble des monomères utilisés, et en particulier comportant au moins 30 % molaire de monomères porteurs d'au moins une fonction acide faible. Avec un tel taux de monomères porteurs d'une fonction acide faible, les inventeurs ont mis en évidence que les propriétés du polymère obtenu étaient réellement dépendantes, d'une part, du taux de neutralisation des fonctions acides des monomères utilisés lors de la polymérisation et, d'autre part, de la concentration totale des monomères dans la phase aqueuse. De manière originale par rapport aux approches proposées dans l'art antérieur qui préconisent de mener la polymérisation avec un taux important de neutralisation des fonctions acide, la demanderesse s'est orientée, dans le cadre de l'invention, vers un procédé de polymérisation en émulsion inverse de polymères présentant un faible taux de neutralisation et, notamment, un taux de neutralisation des fonctions acide présentes d'au plus 20 %.

Dans le cadre de l'invention, la demanderesse propose d'utiliser un tel polymère, obtenu par polymérisation d'une solution aqueuse de monomères en émulsion inverse eau dans huile, dans lequel la polymérisation est réalisée avec une concentration de la totalité des monomères appartenant à la gamme allant de 1,3 mmol à 3,6 mmol par gramme de solution aqueuse. De plus, la demanderesse a démontré qu'une telle gamme de concentrations, contrairement aux concentrations plus importantes notamment utilisées dans l'art antérieur, était compatible avec l'obtention de polymère avec un faible taux de neutralisation des fonctions acide faible présentes et permettait de s'affranchir des problèmes de stabilité constatés dans l'art antérieur,

Dans le cadre de l'invention, le polymère utilisé est obtenu en mettant en oeuvre un procédé de préparation d'un polymère par polymérisation d'une solution aqueuse d'un ou plusieurs monomères en émulsion inverse eau dans huile, dans lequel un ou plusieurs des monomères utilisés comportent au moins une fonction acide, le pourcentage molaire de monomères porteurs d'au moins une fonction acide faible par rapport à l'ensemble des monomères utilisés étant au moins de 30%, caractérisé en ce que :

i) la polymérisation est réalisée avec une concentration de la totalité des monomères en solution aqueuse appartenant à la gamme allant de 1,3 mmol à 3,6 mmol par gramme de solution aqueuse,

ii) lors de la polymérisation, au plus 20% des fonctions acide présentes sur les monomères utilisés possédant au moins une fonction acide se trouvent sous forme neutralisée.

En particulier, lors de la polymérisation, au plus 10%, de préférence au plus 5%, et préférentiellement au plus 2%, des fonctions acide présentes sur les monomères utilisés possédant au moins une fonction acide se trouvent sous forme neutralisée, ce qui permet d'obtenir des propriétés épaississantes encore plus avantageuse. Selon un mode de réalisation particulier, 100% des fonctions acide présentes sur les monomères utilisés se trouvent sous forme acide libre, lors de la polymérisation.

Dans le cadre de l'invention, de manière optimale, la polymérisation est réalisée avec une concentration totale en monomères présents dans la solution aqueuse appartenant à la gamme allant de 1,7 à 3,3 mmol par gramme de solution aqueuse. Dans le cadre de l'invention, les concentrations en monomères sont données par rapport à la masse totale de solution aqueuse (également nommée phase aqueuse), c'est-à-dire masse de monomères comprise.

Notamment, il est donc possible de mener la polymérisation avec les combinaisons suivantes :

- une concentration de la totalité des monomères en solution aqueuse appartenant à la gamme allant de 1,3 mmol à 3,6 mmoi par gramme de solution aqueuse, avec au plus 20%, avantageusement au plus 10%, de préférence au plus 5%, et préférentiellement au plus 2%, voire 0% des fonctions acide présentes sur les monomères possédant au moins une fonction acide qui se trouvent sous forme neutralisée,
- une concentration de la totalité des monomères en solution aqueuse appartenant à la gamme allant de 1,7 mmol à 3,3 mmol par gramme de solution aqueuse, avec au plus 20%, avantageusement au plus 10%, de préférence au plus 5%, et préférentiellement au plus 2%, voire 0% des fonctions acide présentes sur les monomères possédant au moins une fonction acide qui se trouvent sous forme neutralisée.

Le pourcentage molaire en monomères porteurs d'au moins une fonction acide faible par rapport à l'ensemble des monomères utilisés est préférentiellement au moins de 50%, préférentiellement d'au moins 70%, très préférentiellement d'au moins 80%. De tels pourcentages molaires peuvent être utilisés, avec n'importe laquelle des combinaisons concentration en monomères/taux de neutralisation précédemment citées.

Dans le cadre de l'invention, on réalisera, de préférence, la polymérisation avec des monomères qui possèdent tous au moins une insaturation éthylénique.

De manière préférée, la polymérisation est réalisée avec un seul monomère porteur d'au moins une fonction acide faible dont le pourcentage molaire par rapport à l'ensemble des monomères utilisés est au moins de 30%, qui sous forme libre est choisi parmi l'acide acrylique, l'acide méthacrylique, l'acide itaconique, l'acide crotonique, l'acide maléique et l'acide fumarique. Le monomère porteur d'au moins une fonction acide faible est très préférentiellement l'acide acrylique sous forme libre ou avec un taux de neutralisation conforme à l'invention. Il est également possible d'utiliser plusieurs monomères porteurs d'au moins une fonction acide faible, notamment choisis parmi ceux précédemment listés, dont le pourcentage molaire total par rapport à l'ensemble des monomères utilisés est au moins de 30%. De préférence, l'un de ces monomères est l'acide acrylique sous forme libre ou avec un taux de neutralisation conforme à l'invention.

La polymérisation peut être réalisée avec au moins un monomère porteur d'au moins une fonction acide fort. Dans ce cas, la polymérisation est, de préférence, réalisée avec une concentration en monomères porteurs d'au moins une fonction acide fort par rapport à l'ensemble des monomères utilisés de moins de 50%, et préférentiellement de moins de 30%. La polymérisation peut, par exemple, être menée avec un monomère porteur d'au moins une fonction acide fort qui sous forme libre est choisi parmi les acides acrylamidoalkylsulfoniques, tel que l'acide 2-acrylamido-2-méthylpropane sulfonique (ATBS). Dans ce cas, la polymérisation peut, par exemple, être menée avec une combinaison acide acrylique/ATBS ou acide acrylique/ATBS/acrylamide, les monomères acide pouvant se trouver sous forme libre ou avec un taux de neutralisation conforme à l'invention.

Dans le cadre de l'invention, il a été constaté qu'en sélectionnant une concentration en monomères appartenant à la gamme allant de 1,3 mmol à 3,6 mmol par gramme de solution aqueuse pour réaliser la réaction de polymérisation en émulsion inverse, il était possible de préparer des émulsions inverses de polymères porteurs de fonction acide avec un faible taux de neutralisation, voire aucune neutralisation, qui soient stables, c'est-à-dire sans l'observation d'un phénomène rapide de précipitation. De surcroit, il a été démontré qu'une telle gamme de concentrations, contrairement aux concentrations plus importantes notamment utilisées dans l'art antérieur, associée à une faible neutralisation des fonctions acides présentes, permettait d'obtenir des polymères procurant une efficacité épaississante, après une étape de neutralisation au moins partielle, supérieure aux polymères de l'art antérieur obtenus par polymérisation en émulsion inverse. De plus, il a été mis en évidence que ces polymères étaient plus résistants aux électrolytes et que leur utilisation dans la fabrication de compositions cosmétiques ou dermatologiques permettait de réduire la baisse de viscosité due à la présence d'électrolytes, Leur efficacité épaississante est donc améliorée et les compositions obtenues offre un aspect attrayant.

Par « monomère porteur d'au moins une fonction acide », on entend un monomère porteur d'une ou plusieurs fonction(s) acide libre ou neutralisée (c'est-à-dire salifiée par action d'une base). Le terme « fonction acide », sans plus de précision désigne donc à la fois les fonctions acide sous forme libre et sous forme neutralisée. Lorsqu'un monomère comporte plus d'une fonction acide, il est possible de n'avoir qu'une partie des fonctions acide sous forme neutralisée, La ou les fonctions acide présente(s) peu(ven)t être une fonction acide faible ou acide fort. En général, les monomères utilisés ne comporteront que des fonctions acide faible ou que des fonction acide fort, et le plus souvent, des monomères porteurs d'une seule fonction acide seront utilisés. Les mêmes définitions et préférences s'appliquent aux unités monomériques présentes sur le polymère obtenu.

A titre d'exemple de monomère porteur d'au moins une fonction acide faible sous forme libre, du type -COOH, on peut citer l'acide acrylique, l'acide méthacrylique, l'acide itaconique, l'acide crotonique, qui comportent tous une seule fonction acide faible, et l'acide maléique et l'acide fumarique qui comportent, quant à eux, deux fonctions acide faible,

A titre d'exemple de monomère porteur d'une fonction acide fort sous forme libre, on peut citer les monomères porteurs d'une fonction acide phosphonique ou acide sulfonique tels que les acides acrylamidoalkylsulfoniques tel que l'acide 2-acrylamido-2-méthylpropane sulfonique.

Sous leur forme neutralisée, les fonctions acides sont sous forme anionique avec un contre-ion ou cation dépendant de la base utilisée pour la neutralisation, par exemple du type $Na^+$ quand la soude est utilisée ou encore $NH4^+$ quand l'ammoniaque est utilisée. De manière classique, le contrôle du nombre de fonctions acide sous forme neutralisée est assuré par le choix du pH de la solution aqueuse de monomères qui sera ajusté en fonction du pKa des fonctions acide présentes.

La polymérisation peut mettre en jeu un seul type de monomère, alors choisi parmi les monomères porteurs d'au moins une fonction acide faible ou différents types de monomères dont un au moins est porteur d'au moins une fonction acide faible, avec une proportion des fonctions acide présentes sur les monomères utilisés, et donc sur le copolymère obtenu, sous une forme neutralisée qui est inférieure ou égale à 20%. En particulier, en plus du ou des unités monomériques porteuses d'au moins une fonction acide faible précédemment décrit, le polymère obtenu peut contenir d'autres unités monomériques telles que des unités monomériques porteuses d'au moins une fonction acide fort, des unités monomériques neutres (ou non-ioniques), des unités monomériques cationiques et/ou encore des unités monomériques à caractère hydrophobes. Quel que soit le cas, les conditions de formation de la phase aqueuse et de polymérisation sont telles que les fonctions acide des monomères mis en jeu restent majoritairement sous forme libre, et ne soient pas neutralisées par formation d'une forme salifiée, ou faiblement neutralisées avec un taux de neutralisation limité inférieur ou égal à 20%. Lorsqu'une neutralisation inférieure ou égale à 20% a lieu, elle est en général menée dans la phase aqueuse, par ajout d'une quantité de base appropriée. Une base telle que la soude ou l'ammoniaque pourra être utilisée.

Notamment, la réaction de polymérisation peut être réalisée avec au moins un monomère neutre choisi parmi l'acrylamide, le méthacrylamide, le N,N-diméthylacrylamide, le N-vinylméthylacétamide, le N-vinyiformamide, l'acétate de vinyle, le diacétoneacrylamide, le N-isopropyl acrylamide, le N-[2-hydroxy-1,1-bis(hydroxyméthyl) éthyl] propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle), la vinylpyrrolidone, ou d'autres esters acryliques, ou d'autres esters à insaturation éthylénique. Par exemple, la polymérisation peut être réalisée avec de 30 à 99% molaire d'au moins un monomère possédant une ou plusieurs fonction(s) acide faible et de 1 à 70% molaire d'au moins un monomère neutre. La polymérisation peut, par exemple, être menée avec une combinaison acide acrylique/acrylamlde, l'acide acrylique étant sous forme neutre ou avec un taux de neutralisation conforme à l'invention.

Il est également possible de réaliser une copolymérisation avec au moins un monomère cationique. A titre d'exemple de monomères cationiques, on peut citer les sels de diallyldialkyl ammonium comme le chlorure de diallyl diméthyl ammonium (DADMAC) ; les sels acidifiés ou quaternisés d'acrylates et méthacrylates de dialkylaminoalkyle, en particulier d'acrylate de dialkylaminoéthyle (ADAME) et de méthacrylate de dialkylaminoéthyle (MADAME) ; les sels acidifiés ou quaternisés de dialkylaminoalkylacrylamides ou méthacrylamides, comme par exemple le méthacrylamido-propyl triméthyl ammonium chlorure (NAPTAC), l'acrylamido-propyl triméthyl ammonium chlorure (APTAC) et les produits de Mannich comme les dialkylamitiométhylacrylamides quaternisés.

Les sels acidifiés sont obtenus par les moyens connus de l'homme de métier, et notamment par protonation. Les sels quaternisés sont également obtenus par les moyens connus de l'homme du métier notamment, par réaction avec le chlorure de benzyle, le chlorure de méthyle (MeCl), les chlorures d'aryle, d'alkyle, ou le diméthylsulfate.

Il est également possible de réaliser une copolymérisation avec au moins un monomère à caractère hydrophobe. A titre d'exemple de monomères à caractère hydrophobes, on peut citer l'acide undecanoique acrylamide, l'acide undodécyl méthyl acrylamide, les dérivés d'acide acrylique comme les alkyles acrylates ou methacrylates comme par exemple le methacrylate de béhényl 25 éthoxylé. Dans ce cas, le pourcentage molaire en monomères à caractère hydrophobe par rapport à l'ensemble des monomères utilisés est, de préférence, de moins de 10%, et généralement compris entre 0,001% et 7%.

Selon une première variante du procédé selon l'invention, tous les monomères porteurs d'au moins une fonction acide utilisés pour mener la polymérisation sont des monomères porteurs d'au moins une fonction acide faible.

Selon une seconde variante du procédé selon l'invention, la polymérisation est réalisée avec au moins un monomère porteur d'au moins une fonction acide fort, en plus d'au moins un monomère porteur d'au moins une fonction acide faible. Dans ce cas, le pourcentage molaire en monomères porteurs d'au moins une fonction acide fort par rapport à l'ensemble des monomères utilisés est préférentiellement de moins de 50%, très préférentiellement de moins de 30%,

Les copolymères obtenus selon le procédé de l'invention peuvent notamment être formés d'une combinaison d'au moins une unité monomérique porteuse d'au moins une fonction acide faible et d'au moins une unité monomérique porteuse d'au moins une fonction acide fort, et notamment correspondre à un copolymère acide acrylique/ATBS, ces monomères acide étant sous forme neutre ou avec un taux de neutralisation conforme à l'invention ; d'une combinaison d'au moins une unité monomérique porteuse d'au moins une fonction acide faible avec au moins une unité monomérique neutre et éventuellement au moins une unité monomérique porteuse d'au moins une fonction acide fort, et notamment correspondre à un copolymère acide acrylique/acrylamide ou à un copolymère acide acrylique/ATBS/acrylamide, l'acide acrylique et l'ATES étant sous forme neutre ou avec un taux de neutralisation conforme à l'invention ; d'une combinaison d'au moins une unité monomérique porteuse d'au moins une fonction acide faible avec au moins une unité monomérique cationique et éventuellement au moins une unité monomérique porteuse d'au moins une fonction acide fort ; ou encore d'une combinaison d'au moins une unité monomérique porteuse d'au moins une fonction acide faible avec au moins une unité monomérique neutre et au moins un monomère cationique et éventuellement au moins une unité monomérique porteuse d'au moins une fonction acide fort.

Dans le procédé de polymérisation en émulsion inverse utilisé dans le cadre de l'invention, les monomères sont mis en solution aqueuse. Cette solution aqueuse correspond à la phase aqueuse de l'émulsion inverse. Dans le cadre de l'invention, dans la solution aqueuse utilisée pour la polymérisation, au plus 20% des fonctions acide présentes sur les monomères possédant au moins une fonction acide se trouvent sous forme neutralisée.

Il est possible d'utiliser un agent de transfert, autrement appelé agent limitateur de chaîne. L'utilisation d'un agent de transfert est particulièrement avantageuse pour contrôler le poids moléculaire du polymère obtenu. A titre d'exemple, d'agent de transfert, on peut citer le méthanol, l'isopropanol, l'hypophosphite de sodium, le 2-mercaptoéthanol, le méthallysulfonate de sodium et leur mélange, L'homme du métier ajustera, de manière connue, les quantités utilisées d'agent de ramification et éventuellement d'agent de transfert, en fonction de s'il souhaite obtenir un polymère branché ou réticulé et de la viscosité souhaitée,

De manière plus détaillée, le procédé utilisé dans le cadre de l'invention comprend les étapes suivantes :

a) Disposer d'une solution aqueuse du ou des monomères sélectionnés, dite phase aqueuse,

b) Emulsionner ladite solution aqueuse dans une phase non miscible à l'eau, dite phase huile,

c) Mener la réaction de polymérisation.

Bien entendu, la solution aqueuse de l'étape a) présente une concentration totale en monomères, un pourcentage molaire de monomères porteurs d'au moins une fonction acide faible par rapport à l'ensemble des monomères utilisés et un taux de neutralisation des fonctions acide présentes sur les monomères possédant au moins une fonction acide conformes au procédé décrit dans le cadre de l'invention.

De manière classique, l'étape b) d'émulsification de la phase aqueuse dans la phase huile, se fera de préférence par ajout de la phase aqueuse sur la phase huile maintenue sous agitation.

En général, la réaction de polymérisation est réalisée en présence d'un agent émulsionnant eau dans huile. Ce dernier est le plus souvent introduit dans la phase huile dans laquelle la solution aqueuse est émulsionnée. Par agent émulsifiant du type eau dans huile (E/H), on entend un agent émulsifiant possédant une valeur HLB suffisamment faible pour fournir des émulsions eau dans huile, et notamment une valeur HLB inférieure à 10.

La valeur de HLB est calculée selon la relation suivante :

$$HLB = (\% \text{ en poids de la partie hydrophile}) / 5$$

Le pourcentage en poids de la partie hydrophile étant le rapport entre le poids moléculaire de la partie hydrophile sur le poids moléculaire total de la molécule,

A titre d'exemple de tels agents émulsifiants eau dans huile, on peut citer les polymères tensioactifs tels que les polyesters de poids moléculaire compris entre 1000 et 3000 g/mol, produits de la condensation entre un acide poly(isobutènyl) succinique ou son anhydride et un polyéthylène glycol, les copolymères à blocs de poids moléculaire compris entre 2500 et 3500 g/mol, par exemple ceux commercialisés sous les noms HYPERMER®, les extraits de sorbitan, comme le monooléate de sorbitan, l'isostéarate de sorbitan ou le sesquioléate de sorbitan, certains esters de sorbitan polyéthoxylés, comme le monooléate de sorbitan pentaéthoxylé ou l'isostéarate de sorbitan pentaéthoxylé, ou encore l'alcool oléocétylique diéthoxylé, l'acrylate de lauryle tétraéthoxylé.

Dans le procédé de polymérisation en émulsion inverse, la solution aqueuse contient le ou les monomère(s) et éventuellement l'agent de ramification et l'agent de transfert. Elle peut également contenir des agents complexants comme l'éthylène diamine ou l'acide éthylène diamine tétraacétique.

Le plus souvent, la réaction de polymérisation de l'étape c) est amorcée par Introduction dans l'émulsion formée à l'étape b) d'un initiateur de radicaux libres. A titre d'exemple d'agent initiateur de radicaux libres, on peut citer les couples oxydant-réducteur avec parmi les oxydants l'hydroperoxyde de cumène ou le butylhydroxypéroxyde tertiaire, et parmi les réducteurs les persulfates tels que le métabisulfite de sodium et le sel de Mohr. Des composés azoïques tels que le 2,2'-azobis(isobutyronitrile) et le chlorhydrate de 2,2'-azobis(2amidinopropane) peuvent également être utilisés.

De manière classique, la polymérisation est généralement menée de manière isotherme, adiabatique ou à température contrôlée. C'est-à-dire que l'on maintient la température constante généralement entre 10 et 50°C (isotherme), ou alors on laisse la température augmenter naturellement (adiabatique) et dans ce cas on démarre généralement la réaction à une température inférieure à 10°C et la température finale est généralement supérieure à 50°C, ou enfin on contrôle la montée en température pour avoir une courbe de température entre celle de l'isotherme et celle de l'adiabatique.

Il est possible d'introduire à la fin de la réaction de polymérisation. un ou plusieurs agents émulsifiants huile dans eau, de préférence à une température inférieure à 50°C.

Par agent émulsifiant du type huile dans eau (H/E), on entend un agent émulsifiant possédant une valeur HLB suffisamment élevée pour fournir des émulsions huile dans l'eau et notamment une valeur HLB supérieure à 10. A titre d'exemple de tels agents émulsifiants huile dans eau, on peut citer les esters de sorbitan éthoxylés comme l'oléate de sorbitan éthoxylé avec 20 équivalents d'oxyde d'éthylène (EO 20), le laurate de sorbitan polyéthoxylé avec 20 moles d'oxyde d'éthylène, l'huile de ricin polyéthoxylé avec 40 moles d'oxyde d'éthylène, l'alcool oléodécylique décaéthoxylé, l'alcool laurique heptaéthoxylé, ou le monostéarate de sorbitan polyéthoxylé avec 20 moles d'oxyde d'éthylène.

Les quantités d'agent(s) émulsifiant(s) introduites sont telles que l'émulsion inverse du polymère obtenue, contiendra généralement de 1% à 10% en masse, et de préférence de 2,5% à 9% en masse, d'agents émulsifiants du type eau dans huile (E/H) et, éventuellement, de 2% à 10% en masse, et de préférence de 2,5% à 6% en masse d'agents émulsifiants du type huile dans eau (H/E).

En général, le rapport massique de la phase aqueuse sur la phase huile est de 50/50 à 90/10,

La phase huile utilisée dans le procédé de polymérisation en émulsion inverse peut être composée, par exemple, d'une huile minérale, notamment commerciale, contenant des hydrocarbures saturés de type paraffinique, isoparaffinique, cycloparaffinique, naphtalique présentant à température ambiante (22°C), une densité entre 0,7 et 0,9 ; d'une huile végétale ; d'une huile de synthèse tel que le polydécène hydrogéné ou le polyisobutène hydrogéné ; d'un ester tel que l'octyl stéarate ou le buthyl oléate ; d'une huile végétale comme le squalane d'origine végétale ; ou d'un mélange de plusieurs de ces huiles.

A la fin de la réaction de polymérisation. il est également possible que l'émulsion obtenue soit diluée ou concentrée. En particulier, il est possible de concentrer par distillation, l'émulsion obtenue ou bien de complètement la sécher, afin d'obtenir une poudre. Une telle concentration ou séchage seront, menée avec ou sans introduction préalable d'agent émulsifiant du type huile dans eau (H/E).

Les émulsions inverses ainsi obtenues peuvent être concentrées, par exemple par distillation. On obtient alors des émulsions inverses dont la concentration en polymère peut être comprise entre 30 et 75% en masse, préférentiellement entre 40 et 65% en masse.

Les polymères obtenus à partir des émulsions inverses soumises ultérieurement à une étape d'isolation, peuvent se trouver sous la forme d'une poudre. Une telle étape d'isolation peut, par exemple, être choisie parmi les techniques de précipitation, de distillation azéotropique et de séchage par atomisation et pulvérisation.

En effet, dans le cadre de l'invention, il est possible de concentrer ou d'isoler le polymère sous la forme d'une émulsion inverse obtenu directement en sortie du procédé de polymérisation en émulsion inverse, sans perdre les propriétés avantageuses des polymères obtenus, Il existe en particulier de nombreux procédés d'obtention de poudre à partir d'émulsions inverses de polymères qui consistent à isoler la matière active des autres constituants de l'émulsion comme par exemple :

- la précipitation dans un milieu non solvant tel que l'acétone, le méthanol ou tout autre solvant polaire dans lequel le polymère n'est pas soluble. Une simple filtration permet alors d'isoler la particule de polymère.
- la distillation azéotropique en présence d'agent agglomérant et de polymère stabilisant permet de conduire à des agglomérats que l'on isole facilement par filtration avant de procéder au séchage de la particule,
- le "spray-drying* ou séchage par atomisation consiste à créer un nuage de fines gouttelettes d'émulsions dans un courant d'air chaud, pendant une durée contrôlée.

Les polymères obtenus après de telles étapes gardent leurs propriétés avantageuses, en termes de capacité épaississantes et en termes de résistance aux électrolytes.

Sans étape de neutralisation additionnelle, dans les polymères obtenus à l'issu du procédé de polymérisation en émulsion inverse ou après une étape de séchage ou de concentration, au plus 20% des fonctions acide présentes sont sous forme neutralisée, de préférence au plus 10%, de manière encore plus préférée au plus 5%, et préférentiellement au plus 2%. Ce faible taux de neutralisation des fonctions acide présentes offre au formulateur une grande souplesse, lui permettant d'ajuster les propriétés du polymère et donc l'effet épaississant souhaité, en ajustant à façon, le taux de neutralisation. Une telle approche permet également au formulateur de choisir la nature de l'agent neutralisant utilisé, compatible avec l'utilisation ciblée.

Pour obtenir l'effet épaississant désiré, la polymérisation est le plus souvent suivie d'une étape de neutralisation, autrement appelée étape de post-neutralisation, d'au moins une partie, voire de la totalité, des fonctions acide libres présentent sur le polymère. Dans le cas où une étape de neutralisation au moins partielle des fonctions acide libres présentes dans le polymère obtenu est menée après la réaction de polymérisation, elle conduit, de préférence, à un pourcentage de neutralisation par rapport à la totalité des fonctions acide présentes sur le polymère de 30 à 100%.

Une telle étape de post-neutralisation peut être réalisée de différentes manières :

- la post-neutralisation peut être faite sur l'émulsion inverse obtenue à l'issue du procédé de polymérisation en émulsion inverse. Généralement cela est le cas, lorsque le fabricant neutralise lui-même le polymère sous forme d'émulsion inverse.

- La post-neutralisation peut être faite sur une solution aqueuse obtenue suite à l'inversion de l'émulsion inverse dans de l'eau. Généralement, cela est le cas lorsque le formulateur met en oeuvre l'émulsion inverse, ou la poudre en découlant, dans une solution aqueuse, appelée solution mère, avant d'ajouter cette dernière dans la composition à épaissir. Il a alors la liberté d'ajuster la concentration en polymère de la solution, le taux de neutralisation et la nature des agents neutralisant

- La post-neutralisation peut aussi être réalisée sur la composition dans laquelle l'émulsion inverse ou la poudre en découlant a été incorporée. De la même manière que le cas précédent, l'utilisateur a la liberté d'ajuster le taux de neutralisation et la nature des agents neutralisant.

La neutralisation est effectuée grâce à une base, de manière similaire à la neutralisation des monomères précédemment décrite dans le cadre du procédé de polymérisation, dont la nature et les quantités sont sélectionnées par l'homme du métier.

Ces polymères ainsi neutralisés offrent de bien meilleures propriétés épaississantes et de résistance aux électrolytes, toutes conditions égales par ailleurs, comparés aux polymères obtenus par polymérisation en émulsion inverse ne satisfaisant pas les conditions de concentration et de neutralisation des monomères telles que définies dans le procédé selon l'invention. Particulièrement après neutralisation, les polymères offrent des propriétés avantageuses par rapport à des polymères constitués des mêmes monomères, mais préparés par polymérisation en émulsion inverse directement à des taux de neutralisation supérieur et/ou à une concentration totale en monomères différente.

De manière avantageuse, les polymères utilisés dans le cadre de l'invention permettent après complète neutralisation des fonctions acide libre présentes, ou du moins neutralisation plus importante, d'épaissir bien plus efficacement des milieux aqueux présents dans des compositions cosmétiques ou dermatologiques.

Les compositions cosmétiques et dermatologiques selon l'invention et leur procédé de préparation, et en particulier l'incorporation des polymères précédemment décrits, vont maintenant être détaillés.

Les compositions cosmétiques ou dermatologiques sont, tout particulièrement destinées à une application topique. Par "application topique", on entend une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable, c'est-à-dire compatible avec les matières kératiniques. Une composition topique selon l'invention, destinée à être appliquée sur la peau ; les cheveux ou les muqueuses de l'homme ou de l'animal, peut consister en une émulsion topique comprenant au moins une phase aqueuse et au moins une phase huile.

La fabrication de compositions cosmétiques ou dermatologiques est largement connue par l'homme de l'art. Elle consiste généralement à combiner une phase aqueuse, une phase huile avec généralement des tensioactifs, et avec d'autres ingrédients tels que des adjuvants et des additifs. Dans les compositions selon l'invention, la phase aqueuse et la phase huile sont émulsionnées pour former une émulsion eau dans huile, ou le plus souvent huile dans eau. La phase huile de l'émulsion topique peut consister en un mélange d'une ou plusieurs huiles.

L'ajout du polymère acrylique épaississant précédemment décrit peut se faire à n'importe qu'elle étape de la fabrication de la composition cosmétique ou dermatologique. La composition cosmétique ou dermatologique comprend de préférence de 0,01% à 10% en masse de polymère acrylique épaississant, et préférentiellement de 0,1 à 5% en masse, ces pourcentages étant donnés par rapport à la masse totale de la composition.

L'étape de neutralisation conduisant à un pourcentage de fonctions acide neutralisées de 30 à 100% par rapport à la totalité des fonctions acide présentes sur le polymère peut se faire avant ou après l'incorporation du polymère dans la composition.

Par ailleurs, les propriétés avantageuses du polymère obtenu par polymérisation en émulsion inverse selon le procédé précédemment décrit conserve ses propriétés avantageuses, qu'il se présente sous la forme d'une émulsion inverse plus ou moins concentrées, d'une poudre ou d'une solution aqueuse. Par conséquent, le polymère acrylique épaississant pourra être introduit dans la composition cosmétique ou dermatologique, sous la forme d'une émulsion inverse, d'une poudre ou sous forme solubilisée, par exemple dans l'eau ou un solvant organique, ou bien sous forme de dispersion aqueuse ou organique. Généralement c'est une forme solubilisée du polymère dans l'eau qui est introduite dans la composition, obtenue soit par inversion d'une émulsion inverse dans l'eau, soit par dissolution d'une poudre dans l'eau. Quelle que soit la forme sous laquelle il est introduit, dans la composition cosmétique ou dermatologique au moment de son utilisation, le polymère se trouvera dans la phase aqueuse dans laquelle il joue son rôle d'épaississant et de stabilisant.

La phase aqueuse peut contenir tous les ingrédients classiquement utilisés dans une composition cosmétique ou dermatologique et, généralement hydrosolubles. La composition cosmétique ou dermatologique contient, de préférence, de 10 à 99% en masse de phase aqueuse, préférentiellement plus de 20% en masse, et très préférentiellement de 30 à 95% en masse, ces pourcentages étant donnés par rapport à la masse totale de la composition,

La phase huile peut contenir tous les ingrédients classiquement utilisés dans une composition cosmétique ou dermatologique et généralement non solubles dans l'eau. La composition cosmétique ou dermatologique contient. de préférence, de 1 à 99% en masse de phase huile, préférentiellement moins de 80% en masse, et très préférentiellement de 5 à 70% en masse, ces pourcentages étant donnés par rapport à la masse totale de la composition.

Dans le cadre de l'invention, hormis la mise en oeuvre du polymère acrylique épaississant objet de l'invention, la phase aqueuse et la phase huile (nommée également huileuse ou grasse) correspondent à ce qui est traditionnellement utilisé dans les compositions cosmétiques et dermatologiques. On pourra notamment se référer à la demande FR2979821 au nom de l'OREAL dont les parties pertinentes sont reprises ci-après.

La composition selon l'invention peut ainsi comprendre une phase dite aqueuse composée d'eau et de composés hydrophiles. Une telle phase peut comprendre, en plus de l'eau, au moins un solvant organique hydrophile comme les alcools et notamment les monoalcools, linéaires ou ramifiés en $C_1$-$C_6$, comme l'éthanol, le tert-butanol, le n-butanol, l'isopropanol ou le n-propanol, et les polyols comme la glycérine, la diglycérine, le propylène glycol, le sorbitol, le pentylène glycol, et les polyéthylène glycols, ou bien encore les éthers de glycols notamment en $C_2$ et des aldéhydes en $C_2$-$C_4$ hydrophiles.

La phase huile, est notamment constituée de corps gras liquides à température ambiante (en particulier à 25°C). Comme corps gras liquides à température ambiante, appelés souvent huiles, utilisables dans l'invention, on peut citer les huiles hydrocarbonées d'origine animale telles que le perhydrosqualène; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le Parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxyles comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol, l'alcool oléique; les huiles fluorées partiellement hydrocarbonées et/ou siliconées; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes; leurs mélanges. Ces huiles peuvent être présentes en une teneur de 0,01 à 90%, et mieux de 0,1 à 85% en masse, par rapport à la masse totale de la composition.

La phase huileuse est, le plus souvent, principalement constituée d'une huile végétale, d'une huile silicone, d'une huile hydrocarbonée fluorée, d'une huile hydrocarbonée, d'une huile minérale, de polyisobutène, d'isohexadécane, d'un triglycéride caprylique/caprique, d'octanoate de cétéaryle, de benzoate d'alkyle en $C_{12}$-$C_{14}$, ou d'un de leur mélange.

La composition selon l'invention peut également comprendre un ou plusieurs solvants organiques, physiologiquement acceptables, qui peuvent être présents en une teneur de 0,1 à 90%, de préférence de 0,5 à 85%, de préférence encore de 10 à 80% en masse, par rapport à la masse totale de la composition, et mieux de 30 à 50%. On peut notamment citer, outre les solvants organiques hydrophiles cités plus haut, les cétones liquides à température ambiante (en particulier à 25°C) tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol; les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle; les éthers liquides à température ambiante (en particulier à 25°C) tels que le diéthyléther, le diméthyléther ou le dichlorodiéthyléther; les alcanes liquides à température ambiante (en particulier à 25°C) tels que le décane, l'heptane, le dodécane, l'isododécane, le cyclohexane; les composés cycliques aromatiques liquides à température ambiante (en particulier à 25°C) tels que le toluène et le xylène; les aldéhydes liquides à température ambiante (en particulier à 25°C) tels que le benzaldéhyde, l'acétaldéhyde et leurs mélanges.

La composition peut également comprendre avantageusement au moins un agent tensioactif qui est généralement présent en une quantité comprise entre 0,81% et 50% en masse par rapport à la masse totale de la composition, de préférence entre 0,1% et 40% en masse, et encore plus préférentiellement entre 0,5% et 30% en masse. Cet agent tensioactif peut être choisi parmi les agents tensioactifs anioniques, amphotères, non ioniques, cationiques ou leurs mélanges. En particulier, la composition comprendra un agent émulsionnant eau dans huile et/ou un agent émulsionnant huile dans eau, de préférence choisis parmi ceux précédemment cités dans le cadre du procédé de polymérisation du polymère acrylique épaississant.

De manière classique, les compositions selon l'invention comprennent :

- au moins un agent actif choisi parmi les agents hydratants, les agents bronzants, les filtres solaires, les émollients, les principes actifs pharmaceutiques, les vitamines, les oligo-éléments, les agents anti-rides ou anti-âge, les polymères de conditionnement de la peau, les extraits botaniques, les agents à visée amincissante, les agents anti-acnéiques, les agents anti-radicalaires, les agents anti-chutes des cheveux, les agents antipelliculaires et les tensioactifs nettoyants.

- Et/ou au moins un additif, et notamment au moins un auxiliaire de formulation, par exemple choisi parmi les agents chélatants, les agents de neutralisation et d'ajustement du pH, les opacifiants, les conservateurs, les agents d'étalement, les émollients, les polymères filmogènes, les antioxydants, les parfums, les agents réfléchissants, les agents coalescents et les mélanges de ceux-ci.

De tels agents actifs et additifs sont bien connus du formulateur de compositions cosmétiques. On pourra notamment se référer à la demande US 2003/0147825 dont les parties pertinentes sont reprises ci-après.

Agents hydratants

Les agents hydratants peuvent être définis comme étant des matières qui absorbent ou libèrent de la vapeur d'eau, en fonction de l'humidité relative de l'environnement (Harry's Cosmetology, Chemical Publishing Company Inc., 1982, page 266). A titre d'exemple d'agent hydratant, on peut citer l'allantoïne; l'acide pyrrolidonecarboxylique et les sels de celui-ci ; l'acide hyaluronique et les sels de celui-ci ; l'acide sorbique et les sels de celui-ci ; l'urée, la lysine, l'arginine, la cystéine, la guanidine, et d'autres acides aminés ; les polyhydroxyalcools tels que la glycérine, le propylèneglycol, l'hexylèneglycol, l'hexanetriol, l'ethoxydiglycol, le diméthiconecopolyol et le sorbitol, ainsi que les esters de ceux-ci ; le polyéthylèneglycol ; l'acide glycolique et les sels glycolate (par exemple d'ammonium et d'alkylammonium quaternaire) ; le chitosane ; les extraits d'aloe-vera ; les extraits d'algue ;le miel et les extraits de celui-ci ; l'inositol ; l'acide lactique et les sels lactate (par exemple d'ammonium et d'alkylammonium quaternaire) ; les sucres et les amidons ; les dérivés du sucre et de l'amidon (par exemple le glucose alcoxylé) ; le D-panthénol ; l'ascorbylphosphate de magnésium ; l'acide kojique ; la lactamide-monoéthandamine ; l'acétamide-monoéthanolamine ; leurs analogues, et les mélanges de ceux-ci. Lorsqu'ils sont utilisés, les agents hydratants constituent typiquement de 1% à 10% en masse de la masse totale de la composition, de préférence de 2% à 8% en masse, et préférentiellement, de 3% à 5% en masse, de la masse totale de la composition.

Emollients

Un émollient peut être défini comme étant une substance qui régule la vitesse et la quantité de reprise d'eau par la peau (Handbook of Cosmetic Science and Technology, Elsevier Science Publishing, 1993, page 175). A titre d'exemple d'émollient qui peut être incorporé dans la composition selon l'invention, on peut citer les huiles minérales, l'acide stéarique ; les alcools gras tels que l'alcool cétylique, l'alcool cétéarylique, l'alcool myristique, l'alcoolbéhénylique et l'alcool layrylique ; l'acétate de cétyle dans de l'alcool lanolinique acétylé ; l'isostéarate disostéaryle ; les esters de guerbert ; les alcools de guerbert ; le stéarate d'octyle ; le benzoate disostéaryle ; le maléate de dicaprylyle ; le triglycéride caprylique ou caprique ; la vaseline ; la lanoline et les dérivés de celle-ci ; le beurre de coco ; le beurre de karité ; la cire d'abeille éthoxylée ; la cire d'abeille et les esters de celle-ci ; les éthoxylates d'esters de silicone ; les éthoxylates d'éthers alcools gras tels que le cétéareth-20, l'oleth-5 et le céteth-5 ; l'huile ou les glycérides d'avocat ; l'huile ou les glycérides de sésame ; l'huile ou les glycérides de carthame ; l'huile ou les glycérides de tournesol ; les huiles de graines botaniques; l'huile et les glycérides d'écorce de palmier ; l'huile et les glycérides d'amande ; les huiles de silicone volatiles ; les émollients non volatils tels les esters d'acides gras et d'alcools gras, les hydrocarbures fortement ramifiés, et analogues ; leurs analogues ; et les mélanges de ceux-ci, Les esters d'acides gras et d'alcools gras englobent l'oléate de décyle, le stéarate de butyle, le myristate de myristyle, le stéaroylstéarate d'octyldodécyle, l'hydroxystéarate d'octyle, l'adipate de di-isopropyle, le myristate d'isopropyle, le palmitate d'éthylhexyle, le néo-pentanoate d'isodécyle, le benzoate d'alcool en $C_{12}$-$C_{15}$, le maléate de diéthylhexyle, le butyéther de PPG-147 et le propionate de myristyléther de PPG-2, l'octanoate de cétéaryle, leurs analogues, et les mélanges de ceux-ci. Les hydrocarbures fortement ramifiés convenables englobent l'isohexadécane, et ses analogues, le polyisobutène hydrogéné, le polyisobutène, et les mélanges de ceux-ci. Sont aussi compris les silicones volatiles, telles que les polydiméthylsiloxanes cycliques ou linéaires, et leurs analogues. Le nombre d'atomes de silicium dans les silicones cycliques peut s'échelonner de 3 à 7 ou de 4 à 5. « Volatile » signifie que la silicone a une pression de vapeur mesurable. On peut trouver une description des silicones volatiles dans Told and Byers, « Volatile Silicone Fluids for Cosmetics », Cosmetics and Toiletries, volume 91, janvier 1976, pages 27-32. D'autres émollients convenables englobent les gommes de polydiméthylsiloxane, les aminosilicones, les phénylsilicones, le polydiméthylsiloxane, le polydiéthylsiloxane, le polyméthylphénylsiloxane, les gommes de polydiméthylsiloxane, les gommes de polyméthylphénylsiloxane, !'amodiméthicone, la triméthylsilylamodiméthicone, les gommes de diphényldiméthylpolysiloxane, et leurs analogues, Lorsque un ou plusieurs sont présents dans la composition, il(s) représente(nt) de 1% à 20% en masse, de la masse totale de la composition, préférentiellement, de 2% à 15% en masse, et de préférence de 3% à 10% en masse, de la masse totale de la composition.

## Principes actifs pharmaceutiques

Le ou les principes actifs pharmaceutiques qui peuvent être incorporés dans la composition selon l'invention peuvent être toute substance, matière ou composé chimique, qui induit un effet pharmacologique local ou systémique souhaité. Ces principes actifs englobent, mais sans y être limités, les agents anti-fongiques, les agents anti-bactériens, les agents anti-inflammatoires, les myorelaxants, les antibiotiques, les agents antiviraux, les analgésiques (par exemple l'ibuprofène, l'acide acétylsalicylique, le naproxène, et leurs analogues), les anti-histaminiques, les agents antipruritiques, les agents antipyrétiques, les agents anesthésiques, les agents diagnostiques, les hormones, les rehausseurs de croissance cutanée, les modulateurs pigmentaires, les agents antiprolifératifs, les agents antipsoriatiques, les rétinoïdes, les médicaments anti-acné (par exemple le peroxyde de benzoyle, le soufre et leurs analogues), les agents antinéopastiques, les agents photothérapeutiques, les agents kératolytiques (par exemple le résorcinol, l'acide salicylique et leurs analogues), et leurs analogues. Lorsque la composition comprend un ou plusieurs principes actifs pharmaceutiques, ils représentent typiquement de 0,1% à 20% en masse de la masse totale de la composition.

## Extraits botaniques

Les extraits botaniques sont définis comme étant des extraits provenant de plantes ou végétaux qui peuvent être obtenus par l'intermédiaire de diverses préparations notamment la teinture, un extrait fluide, un extrait solide, un extrait pulvérulent, une dilution homéopathique, un extrait d'essence, un extrait aqueux, et leurs analogues. Les propriétés de ces préparations sont décrites dans Botanicais A Phytocosmetic Desck Reference, Franck S. D'Amelio, Sr., CRC Press LLC, 1999, page 39. Les extraits botaniques englobent, mais sans y être limités, les extraits des plantes ou végétaux suivants : l'aloe-vera, l'alfalfa, la pomme, l'artichaut, l'avéna, la vinette, la busserole, le pollen d'abeille, l'airelle, la noix noire, le bourrache, la calendule, le piment, la camomille, le caïeu, le concombre, la coriandre, le ginseng, le gingembre, le ginko, le gotukola, le thé vert, le henné, le miel, la châtaigne, les fleurs de jasmin, le chanvre, le jonc odorant, la racine de réglisse, le souci, l'avoine, la fleur d'oranger, la papaye, la mure, le bigorneau, la rose, le cynorhodon, le romarin, le bois de sandale, les herbes marines, la spiruline, l'huile de l'arbre à thé, la noix, le chiendent, l'orme blanc, le yohimbehe, et leurs analogues. Le ou les extraits botaniques lorsqu'ils sont utilisés dans les compositions selon l'invention représentent typiquement de 0,05% à 2% en masse de la masse totale de la composition,

## Filtres solaires

Les filtres solaires lorsqu'ils sont incorporés dans les compositions selon invention seront utilisés en des quantités sûres et efficaces en termes de photoprotection, c'est-à-dire en quantité suffisante pour fournir une photoprotection lorsque la composition est appliquée, mais pas trop élevée au point d'entraîner un effet secondaire tel que des réactions de la peau. Des exemples de filtres solaires englobent ceux présentés dans Segarin et al., Cosmetics Science and Technology chapitre VIII, pages 1890 et suivantes, ainsi que dans 64 Fed. Reg. 27666-27693 (21 mai 1999). A titre d'exemples de filtres solaires, on peut citer l'acide p-aminobenzoïque, ses sels et ses dérivés (les éthyl, isobuthyl, glycéryl-esters ; l'acide p-diméthylaminobenzoïque ; le N,N-diméthylaminobenzoate de 2-éthylhexyle) ; les anthranilates (à savoir les o-aminobenzoates ; les méthyl, octyl, amyl, menthyl, phényl, benzyl, phényléthyl, linalyl, terpinyl et cyclohexényl-esters) ; les salicylates (les octyl, amyl, phényl, benzyl, menthyl, glycéryl et dipropylèneglycol-esters) ; les dérivés de l'acide cinnamique (les éthylhexyl-p-méthoxy, menthyl et benzyl-esters ; le phénylcinnamonitrile ; le pyruvate de butylcinnamoyle) ; les dérivés de l'acide dihydroxycinnamique (l'umbelliférone, la méthylumbélliférone, la méthylacéto-umbelliférone) ; les dérivés de l'acide trihydroxycinnamique (l'esculétine, la méthylesculétine, la daphnétine et les glycosides, l'esculine et la daphnine) ; les hydrocarbures (le diphénylbutadiène, le stilbène) ; la dibenzalacétone et la benzalacétophénone ; les naphtosulfonates (les sels de sodium des acides 2-napthol-3,6-disulfonique et 2-naphtol-6,8-disulfonique) ; l'acide dihydroxynaphtoïque et ses sels ; les o- et p-hydroxybiphényldisulfonates ; les dérivés de coumarine (la 7-hydroxy, la 7-méthyl et la 3-phényl) ; les diazoles (le 2-acéthyl-3-bromoindazole, le phénylbenzoxacole, le méthylnaphtoxazole et divers arylbenzothiazoles) ; les sels de quinine (le bisulfate, le sulfate, le chlorure, l'oléate et le tannate) ; les dérivés de quinoléine (les sels de 8-hydroxyquinoléine et la 2-phénylquinoléine) ; les benzophénones hydroxyméthoxy--substituées ; les acides urique et vilourique ; l'acide tannique et ses dérivés (par exemple l'hexaéthyléther) ; le (butylcarbityl)(6-propylpipéronyl)éther ; l'hydroquinone ; les benzophénones (l'oxybenzone, la sulisobenzone, la dioxybenzone, le benzorésorcinol, la 2,2',4,4'-tétrahydroxybenzophénone, la 2,2'-dhydroxy-4,4'-diméthoxybenzophénone, l'octabenzone, le 4-isopropyldibenzoylméthane, le butylméthoxydibenzoylméthane ; l'octocrylène ; le 4-isopropyldibenzoylméthane) ; les dérivés du camphre tels que le méthylbenzylidène ou le benzylidènecamphre ; leurs analogues, et les mélanges de ceux-ci.

Lorsqu'ils sont utilisés dans les compositions de la présente invention, les filtres solaires représentent de 0,5% à 50% en masse de la masse totale de la composition, de préférence, de 0,5% à 30% en masse, et préférentiellement de 0,5% à 20% en masse, de la masse totale de la composition. La quantité incorporée variera en fonction du filtre solaire choisi et de la quantité souhaitée de facteur de protection solaire (SPF) comme défini dans la demande US 2003/0147825.

Tensioactifs nettoyants

Les tensioactifs nettoyants qui peuvent être incorporés dans la composition selon l'invention englobent une diversité de tensioactifs non ioniques, cationiques, anioniques et zwittérioniques, tels que ceux décrits dans Mc Cutcheon's detergents and Emulsifiers, North American Edition (1996), Allured Publishing Corporation, et dans les brevets américains US-A-3 755 560 ; 4 421 769 ; 4 704 272 ; 4 741 855 ; 4 788 006 et 5 011 681. Des exemples de tensioactifs convenables englobent, mais sans y être limités, les alkyl et alcénylsulfates, les alkyl et alcénylsulfates éthoxylés (ayant, de préférence, un degré moyen d'éthoxylation de 1 à 10) ; les tensioactifs de type succinamate tels que les sulfosuccinamates d'alkyle et les esters dialkyliques de l'acide sulfosuccinique ; les esters d'acide gras neutralisés de l'acide iséthionique ; et les sulfonates d'alkyle et d'alcényle, tels que les sulfonates d'oléfines et les sulfonates de béta-alcoxyalcane ; et leurs analogues. Sont préférés, les alkyl et alcénylsulfates, les alkyl et alcénylsulfates éthoxylés, par exemple les sels de sodium et d'ammonium de sulfates et de sulfates éthoxylés en $C_{12}$-$C_{18}$ (ayant, de préférence, un degré dethoxylation de 1 à 6, et préférentiellement de 1 à 4), tels que le laurylsulfate et le laureth (3,0)-sulfate ; le 3-dodécylaminopropionate de sodium ; les N-alkyltaurines telles que celles préparées par réaction de dodécylamine avec de l'iséthionate de sodium selon US-A-2 658 072 ; les acides (N-alkyl supérieur) aspartiques tels que ceux produits selon US-A-2 438 091 ; et les produits vendus sous la marque de commerce « Miranol » décrits dans US-A-2 528 378, et leurs analogues. D'autres tensioactifs convenables englobent les amphoglycinates d'alkyle en $C_6$-$C_{22}$ et les amphopropionates d'alkyle en $C_6$-$C_{22}$ et de préférence, les amphoglycinates d'alkyle les amphopropionates d'alkyle en $C_8$-$C_{12}$ ; les tensioactifs nettoyants zwittérioniques choisis parmi les composés aliphatiques d'ammonium quaternaire, de phosphonium et de sulfonium en $C_8$-$C_{16}$, qui portent un substituant contenant un groupe anionique de solubilisation dans l'eau, tel qu'un carboxy, un sulfonate, un sulfate, un phosphate, un phosphonate, et analogues, les aminosulfonates d'alkyle, les alkylbétaïnes et les alkylamidobétaïnes, la stéaramidopropyldiméthylamine, le diéthylaminoéthylstéaramide, la diméthylstéaramine, la diméthylsojamine, la sojamine, la myristylamine, la tridécylamine, l'éthylstéarylamine, la N-tallowpropanediamine, la stéarylamine éthoxylée (5 moles d'oxyde d'éthylène)" la dihydroxyéthylstéarylamine, l'arachidylbéhénylamine, et leurs analogues. Lorsque la composition contient un ou plusieurs tensioactifs nettoyants, ceux-ci représentent typiquement de 0,5% à 20% en masse, et préférentiellement de 1% à 12% en masse, de la masse totale de la composition.

Polymères de conditionnement de la peau

A titre d'exemples de polymère de conditionnement de la peau qui peut être incorporé dans la composition, on peut citer la gomme guar quaternisée, les composés cellulosiques quaternisés, le polyquaternium 4, le polyquaternium 7, le polyquaternium 10, le polyquaternium 11, le polyquaternium 39, le polyquaternium 44, et leurs analogues. Lorsque la composition contient un ou plusieurs polymères de conditionnement, ceux-ci représentent typiquement de 0,01% à 3% en masse, de la masse totale de la composition, de préférence, de 0,1% à 2% en masse, et préférentiellement de 0,1% à 1% en masse, de la masse totale de la composition.

Vitamines

A titre d'exemples de vitamine qui peut être incorporée dans la composition, on peut citer la vitamine A, la vitamine B, la biotine, l'acide pantothénique, la vitamine C, la vitamine D, la vitamine E, l'acétate de tocophérol, le palmitate de rétinyle, l'ascorbylphosphate de magnésium, leurs analogues et dérivés. Lorsque la composition contient une ou plusieurs vitamines, elle(s) représente(nt) typiquement de 0,001% à 5% en masse, de la masse totale de la composition totale, de préférence, de 0,01% à 2% en masse, et préférentiellement de 0,1% à 1% en masse, de la masse totale de la composition.

Agents chélatants

A titre d'exemples d'agents chélatants qui peut être incorporé dans la composition, on peut citer l'EDTA (acide éthylènediaminetétraacétique) et les sels de celui-ci tels que l'EDTA disodique ; l'acide citrique et les sels de celui-ci ; les cyclodextrines ; et leurs analogues. Lorsque la composition contient un ou plusieurs agents chélatants, il(s) représente(nt) typiquement de 0,001% à 3% en masse, de la masse totale de la composition, de préférence, de 0,01% à 2% en masse, et préférentiellement de 0,01% à 1% en masse, de la masse totale de la composition.

Agents de neutralisation et agents d'ajustement du pH

Des agents de neutralisation et des agents d'ajustement du pH peuvent être incorporés dans la composition afin d'amener le pH de la composition aux niveaux souhaités. A titre d'exemples d'agents de neutralisation et d'agents d'ajustement du pH on peut citer la triéthanolamine, l'aminométhylpropanol, l'hydroxyde d'ammonium, l'hydroxyde de sodium, d'autres hydroxydes alcalins, les borates, les phosphates, les pyrophosphates, le coco-amine, l'oléilamine, la diisopropanolamine, la diisopropylamine, la dodécylamine, le coco-amine de PEG-15, la morphine, la tétrakis(hydroxypropyl)éthylènediamine, la triamylamine, la triéthanolamine, la triéthylamine, la trométhamine (le 2-amino-2-hydroxyméthyl-1,3-propanediol), l'acide ascorbique et les sels de celui-ci, l'acide sorbique et les sels de celui-ci, l'acide phosphorique et les sels de celui-ci, l'acide citrique et les sels de celui-ci, l'acide lactique et les sels de celui-ci, l'acide glycolique et les sels de celui-ci, l'acide borique et les sels de celui-ci, l'acide acétique et les sels de celui-ci, et leurs analogues. De préférence, les agents de neutralisation et les agents d'ajustement du pH sont utilisés dans la composition de l'invention en une quantité suffisante pour conférer un pH s'échelonnant de 4 à 10. De préférence, les agents d'ajustement du pH sont utilisés en une quantité suffisante pour conférer à la composition un pH s'échelonnant de 4,5 à 8, et préférentiellement de 5 à 7,5.

Opacifiants

A titre d'exemples d'opacifiants, on peut citer les esters d'acide gras de glycol tels que le dibéhénate de glycol, le dioléate de glycol, le distéarate de glycol, le dilallowate de glycol, l'hydroxystéarate de glycol, le montanate de glycol, le palmitate de glycol et le stéarate de glycol ; les acides gras et les mélanges d'acide gras hydrogénés tels que l'acide béhénique, l'acide arachidique, l'acide palmitique, l'acide myristique, l'acide de maïs, l'acide de palme, l'acide d'écorce de palmier, l'acide de coprah hydrogéné, l'acide de menhaden hydrogéné, l'acide de palme hydrogéné, l'acide de suif hydrogéné, les esters d'acide gras alcoxylés ; la silice ; les alcanolamides tels que le béhénamide, le linoléamide et le stéaramide ; le talc ; le nylon ; les alcools d'acide gras tels que l'alcool arachidylique, l'alcool béhénique, l'alcool stéarylique, l'alcool cétylique et l'alcool myristylique ; les cires et les huiles ; le kaolin ; le silicate de magnésium ; et leurs analogues. Le ou les opacifiants, lorsqu'ils sont présents dans la composition, représentent typiquement de 0,1% à 8% en masse de la masse totale de la composition, de préférence, de 0,5% à 6% en masse, et préférentiellement de 1% à 5% en masse, de la masse totale de la composition de la présente invention.

Conservateurs

A titre d'exemples de conservateurs, on peut citer la polyméthoxyoxazolidine bicyclique, le méthylparabène, le propylparabène, l'éthylparabène, le butylparabène, l'acide benzoïque et les sels de l'acide benzoïque, le benzyltriazole, la DMDM-hydantoïne (aussi connue sous le nom 1,3-diméthyl-5,5-diméthyl-hydantoïne), l'imidaolidinylurée, le phénoxyéthanol, le phénoxyéthylparabène, la méthylisothiazolinone, la méthylchloroisothiazolinone, la benzoisothiazolinone, le triclosan, l'acide sorbique, les sels de l'acide salicylique, et leurs analogues. Le ou les conservateurs, lorsqu'ils sont présents dans la composition, représentent typiquement de 0,01% à 1,5% en masse de la masse totale de la composition, de préférence de 0,1% à 1% en masse, et préférentiellement de 0,3% à 1% en masse, de la masse totale de la composition.

Agents d'étalement

A titre d'exemples d'agents d'étalement, on peut citer l'hydroxypropylméthylcellulose, les composés cellulosiques modifiés de façon à être hydrophobes, la gomme de xanthane, la gomme d'acacia, la gomme guar, la gomme de graine de caroube, les diméthiconecopolyols ayant divers degrés d'alcoxylation, le silicate d'aluminium-magnésium, le nitrure de bore, le talc, et leurs analogues. Le ou les d'agents d'étalement, lorsqu'ils sont présents dans la composition, représentent typiquement de 0,01% à 1,5% en masse de la masse totale de la composition, de préférence, de 0,1% à 3% en masse, et préférentiellement de 0,1% à 2 % en masse, de la masse totale de la composition.

En outre, l'invention est particulièrement adaptée aux compositions comprenant un électrolyte qui peut être défini comme une substance chimique chargé positivement ou négativement et capable de transporter ou conduire une charge électrique, généralement dans une solution. On les appelle également des composés ioniques et dans le domaine de la formulation de compositions cosmétiques et dermatologiques, des ingrédients ioniques. Ils peuvent être monovalents ou multivalents. Les électrolytes sont principalement des acides, des bases ou des sels. Plus précisément parmi les électrolytes souvent rencontrés dans les compositions cosmétiques et dermatologiques, on peut citer des adjuvants comme les extraits végétaux contenant des ions monovalent ou divalents tels que les acides de fruits, les principes actifs tels que les hydroxy acides pour leur effet anti-âge, les agents hydratants tels que l'acide carboxylique pyrrolidone (PCA), des agents chelatants comme par exemple l'acide éthylène diamine tétraacétique (EDTA), les filtres UV comme l'acide sulfonique phenylbenzimidazole, certains conservateurs ou encore des sels comme par exemple le sel d'alun.

Une composition topique selon l'invention peut être destinée à une utilisation cosmétique, excluant tout traitement thérapeutique, ou être utilisée en tant que médicament destiné au traitement des maladies de la peau et des muqueuses. Dans ce dernier cas, la composition topique comporte alors au moins un principe pharmaceutiquement actif, par exemple choisi parmi les agents anti-fongiques, les agents anti-bactériens, les agents anti-inflammatoires, les myorelaxants, les antibiotiques, les agents antiviraux, les analgésiques (par exemple l'ibuprofène, l'acide acétylsalicylique, le naproxène, et leurs analogues), les anti-histaminiques, les agents antipruritiques, les agents antipyrétiques, les agents anesthésiques, les agents diagnostiques, les hormones, les rehausseurs de croissance cutanée, les modulateurs pigmentaires, les agents antiprolifératifs, les agents antipsoriatiques, les rétinoïdes, les médicaments anti-acné (par exemple le peroxyde de benzoyle, le soufre et leurs analogues), les agents antinéopastiques, les agents photothérapeutiques, les agents kératomiques (par exemple le résorcinol, l'acide salicylique et leurs analogues), et leurs analogues. Bien entendu, l'homme du métier adaptera la quantité de ce ou ces éventuels composés complémentaires, pour obtenir l'effet souhaité.

Les polymères acryliques épaississant utilisés dans le cadre de l'invention peuvent être utilisés dans les applications décrites dans la demande EP 1 710 259 au nom de SEPPIC. Aussi, ils pourront être associés avec des esters d'acides gras et de sucre, pour former des compositions pour le traitement du cheveux ou de la peau telles que celles décrites dans EP 0 603 019 , ou encore dans les shampooings ou après-shampooings tels que décrits et revendiqués dans WO 92/21316 ou enfin en association avec un homopolymère anionique tels que le CARBOPOL pour former des produits de traitement des cheveux comme ceux décrits dans DE 195 23596.

Les polymères acryliques épaississant utilisés dans le cadre de l'invention sont également compatibles avec les principes actifs tels que par exemple, les agents auto-bronzants comme le dihydroxy-acétone (DHA) ou les agents anti-acné; elle peut donc être introduite dans des compositions auto-bronzantes comme celles revendiquées dans EP 0 715 845, EP 0 604 249, EP 0576188 ou dans WO 93/07902.

Les polymères acryliques épaississant utilisés dans le cadre de invention sont également compatibles avec les dérivés N-acylés d'aminoacides, et peuvent donc être utilisés dans des compositions apaisantes notamment pour peau sensible, telles que celles décrites ou revendiquées dans WO 92/21318, WO 94/27561, ou WO 98/09611.

Les compositions cosmétiques selon l'invention peuvent se présenter sous la forme d'un produit de soin et/ou de maquillage de la peau du corps ou du visage, des lèvres et des cheveux, d'un produit solaire ou autobronzant, d'un produit capillaire tels que les shampooings, gels, lotions de mise en plis, lotions pour le brushing.

Dans un mode de réalisation préféré, les compositions conformes à l'invention peuvent être utilisées pour le lavage ou le traitement, en particulier cosmétique, des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

L'invention a donc encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Les exemples ci-après permettent d'illustrer l'invention, mais n'ont aucun caractère limitatif.

**I. Exemples de préparation d'homopolymère à base d'acide acrylique / acrylate de sodium**

Exemple 1 :

On charge dans un bécher de 1 L sous agitation magnétique les ingrédients de la phase aqueuse:

- 150 g d'acide acrylique glacial
- 605 g d'eau déionisée
- 0,023 g d'hypophosphite de sodium (150 ppm/masse totale de monomères)
- 0,10 g de diéthylènetriaminepentacétate de sodium
- 0,075 g de méthylènebisacrylamide (500 ppm/masse totale de monomères)
- 0,15 g de bromate de sodium

Ensuite dans un réacteur en verre de 1 L, sous agitation mécanique, la phase organique est préparée avec :

- 102 g d'hydrocarbure aliphatique (Isopar L)
- 98 g d'huile minérale blanche (Marcol 152)
- 20 g de monooléate de sorbitol
- 25 g de stabilisant polymérique (Hypermer 1083).

La phase aqueuse est transférée progressivement dans la phase organique. La pré-émulsion ainsi formée est alors soumise à fort cisaillement pendant 1 minute (Ultra Turrax, IKA).

L'émulsion inverse est alors dégazée pendant 30 minutes grâce à un simple barbotage d'azote.

On ajoute alors une solution aqueuse contenant 1,0 % en masse de métabisulfite de sodium à un débit de 2,5 ml/h pendant une durée de 1h30. Une fois la température maximum atteinte, on maintient la température du mélange réactionnel pendant 60 minutes avant refroidissement.

Enfin, on ajoute vers 30°C, 40 g d'alcool tridécylique éthoxylé (6 moles).

Exemple 2 :

On charge dans un bécher de 1 L sous agitation magnétique les ingrédients de la phase aqueuse :

- 175 g d'acide acrylique glacial
- 580 g d'eau déionisée
- 0,026 g d'hypophosphite de sodium (150 ppm/masse totale de monomères)
- 0,10 g de diéthylènetriaminepentacétate de sodium
- 0,087 g de méthylènebisacrylamide (500 ppm/masse totale de monomères)
- 0,15 g de bromate de sodium

Ensuite, pour la préparation de la phase organique et le reste du procédé de préparation, on procède conformément à l'exemple 1.

Exemple 3 :

On charge dans un bécher de 1 L sous agitation magnétique les ingrédients de la phase aqueuse :

- 100 g d'acide acrylique glacial
- 655 g d'eau déionisée
- 0,015 g d'hypophosphite de sodium (150 ppm/masse totale de monomères)
- 0,10 g de diéthylènetriaminepentacétate de sodium
- 0,05 g de méthylènebisacrylamide (500 ppm/masse totale de monomères)
- 0,15 g de bromate de sodium

Ensuite, pour la préparation de la phase organique et le reste du procédé de préparation, on procède conformément à l'exemple 1.

Exemple 4 : Neutralisation 3,5%/concentration 2,76

On réalise le même procédé que dans l'exemple 1 en ajoutant dans la phase aqueuse 5,83 g de solution d'hydroxyde de sodium 50%, tout en conservant le même poids de phase aqueuse en ajustant la quantité d'eau désionisée.

Exemple 5 : Neutralisation 19%/concentratlon 3,5

On charge dans un bécher de 1 L sous agitation magnétique les ingrédients de la phase aqueuse :

- 190 g d'acide acrylique glacial
- 40 g de solution d'hydroxyde de sodium 50%
- 525 g d'eau déionisée
- 0,028 g d'hypophosphite de sodium (150 ppm/masse totale de monomères)
- 0,10 g de diéthylènetriaminepentacétate de sodium
- 0,095 g de méthylènebisacrylamide (500 ppm/masse totale de monomères)
- 0,15 g de bromate de sodium

Ensuite, pour la préparation de la phase organique et le reste du procédé de préparation, on procède conformément à l'exemple 1.

Exemple 6

On charge dans un bécher de 1 L sous agitation magnétique les ingrédients de la phase aqueuse:

- 150 g d'acide acrylique glacial
- 605 g d'eau déionisée
- 0,10 g de diéthylènetriaminepentacétate de sodium
- 0,225 g de méthylènebisacrylamide (1500 ppm/masse totale de monomères)
- 0,15 g de bromate de sodium

Ensuite dans un réacteur en verre de 1 L, sous agitation mécanique, la phase organique est préparée avec ;

- 102 g d'hydrocarbure aliphatique (Isopar L)
- 98 g d'huile minérale blanche (Marcol 152)
- 20 g de monooléate de sorbitol
- 25 g de stabilisant polymérique (Hypermer 1083),

La phase aqueuse est transférée progressivement dans la phase organique. La pré-émulsion ainsi formée est alors soumise à fort cisaillement pendant 1 minute (Ultra Turrax, IKA).
L'émulsion inverse est alors dégazée pendant 30 minutes grâce à un simple barbotage d'azote.
On ajoute alors une solution aqueuse contenant 1,0 % en masse de métabisulfite de sodium à un débit de 2,5 ml/h pendant une durée de 1h30. Une fois la température maximum atteinte, on maintient la température du mélange réactionnel pendant 60 minutes avant refroidissement.
Enfin, on ajoute vers 30°C, 40 g d'alcool tridécylique éthoxylé (6 moles).

Exemple comparatif 1 :

On charge dans un bécher de 1 L sous agitation magnétique les ingrédients de la phase aqueuse :

- 50 g d'acide acrylique glacial
- 705 g d'eau déionisée
- 0,075 g d'hypophosphite de sodium (150 ppm/masse totale de monomères)
- 0,10 g de diéthylènetriaminepentacêtate de sodium
- 0,043 g de méthylènebisacrylamide (860 ppm/masse totale de monomères)
- 0,15 g de bromate de sodium

Ensuite, pour la préparation de la phase organique et le reste du procédé de préparation, on procède conformément à l'exemple 1.

Exemple comparatif 2 :

On charge dans un bêcher de 1 L sous agitation magnétique les ingrédients de la phase aqueuse :

- 199 g d'acide acrylique glacial
- 115 g de solution d'hydroxyde de sodium 50%
- 441 g d'eau déionisée
- 0,03 g d'hypophosphite de sodium (150 ppm/masse totale de monomères)
- 0,10 g de diéthylènetriaminepentacétate de sodium
- 0,15 g de méthylènebisacrylamide (750 ppm/masse totale de monomères)
- 0,15 g de bromate de sodium

Ensuite, pour la préparation de la phase organique et le reste du procédé de préparation, on procède conformément à l'exemple 1,

Exemple comparatif 3 :

On charge dans un bécher de 1 L sous agitation magnétique les Ingrédients de la phase aqueuse :

- 199 g d'acide acrylique glacial
- 556 g d'eau déionisée
- 0,03 g d'hypophosphite de sodium (150 ppm/masse totale de monomères)
- 0,10 g de diéthylènetriaminepentacétate de sodium
- 0,1 g de méthylènebisacrylamide (500 ppm/masse totale de monomères)
- 0,15 g de bromate de sodium

Ensuite, pour la préparation de la phase organique, on procède conformément à l'exemple 1.
La phase aqueuse est transférée progressivement dans la phase organique. La pré-émulsion ainsi formée est alors soumise à fort cisaillement pendant 1 minute (Ultra Turrax, IKA).
L'émulsion inverse est alors dégazée pendant 30 minutes grâce à un simple barbotage d'azote.
On ajoute alors une solution aqueuse contenant 1,0 % en masse de métabisulfite de sodium à un débit de 2,5 ml/h. Immédiatement après le début de l'ajout de cette solution réductrice, l'émulsion est déstabilisée puis coagule, La polymérisation est impossible, le système n'est pas stable.

Exemple comparatif 4 :

On charge dans un bécher de 1 L sous agitation magnétique les ingrédients de la phase aqueuse :

- 150 g d'acide acrylique glacial
- 83 g de solution d'hydroxyde de sodium 50%
- 522 g d'eau déionisée
- 0,023 g d'hypophosphite de sodium (150 ppm/masse totale de monomères)
- 0,10 g de diéthylènetriaminepentacétate de sodium
- 0,75 g de méthylènebisacrylamide (500 ppm/masse totale de monomères)
- 0,15 g de bromate de sodium

Ensuite, pour la préparation de la phase organique et le reste du procédé de préparation, on procède conformément à l'exemple 1.

Caractérisation des polymères

Procédure : Mesure de la viscosité de la solution aqueuse de polymère à iso-concentration [0,16 % en masse]
Dans un bêcher de 400 ml on introduit 250 g d'eau déionisée, puis sous agitation mécanique (tripâle - 500 tours par minute), on ajoute progressivement la quantité d'émulsion inverse désirée pour obtenir une solution contenant 0,16% en masse de polymère actif, Le pH est ensuite ajusté à 7 +/-0,1 avec de l'hydroxyde de sodium. A ce pH, 100% des fonctions acide présentes sur le polymère sont neutralisées. La solution est laissée 15 minutes sous agitation puis 5 minutes au repos, La viscosité est alors mesurée à l'aide d'un viscosimètre Brookfield de type RVT avec le module 4 et vitesse de rotation 20 tours par minute.

Les résultats sont consignés dans le **Tableau 1.**

**Tableau 1**

| Exemple | NFA | CM | Viscosité 0,16% dans l'eau (cps) |
|---|---|---|---|
| 1 | 0% | 2,8 | 6500 |
| 2 | 0% | 3,2 | 4000 |
| 3 | 0% | 1,8 | 6200 |
| 4 | 3,5% | 2,8 | 6500 |
| 5 | 19% | 3,5 | 2500 |
| 6 | 0% | 2,8 | 15000 |
| Comparatif 1 | 0% | 0,9 | 1700 |
| Comparatif 2 | 52% | 3,7 | 500 |
| Comparatif 3 | 0% | 3,7 | Emulsion non stable |
| Comparatif 4 | 50% | 2,8 | 1500 |
| ET75 | 50% | 3,8 | 50 |

NFA : Neutralisation des fonctions acide (%) à l'Issue de la polymérisation CM : Concentration en monomère en mmol/g de phase aqueuse ET75 est une émulsion Inverse commerciale d'homopolymère d'acide acrylique dont 50% des fonctions acide ont été neutralisées avant la polymérisation.

Les polymères utilisés dans le cadre de l'Invention obtenus par le procédé de polymérisation en émulsion inverse présentent un effet épaississant bien supérieur aux polymères obtenus par des procédés d'émulsion inverse ne satisfaisant pas aux conditions de % de neutralisation avant polymérisation et concentration en monomères,

Les polymères obtenus conformément à l'invention sont très efficaces à très faible concentration.

La résistance aux électrolytes est évaluée en mettant en oeuvre ces mêmes polymères dans de l'eau déionisée et en présence d'un électrolyte, l'acide éthylène diamine tétraacétique (EDTA).

Les polymères sont comparés entre eux et à d'autres polymères épaississants disponibles commercialement Il s'agit du Sepigel® 305 (SEPPIC) et du Novemer® EC2 (Noveon) qui sont des émissions inverses, et du Carbopol® 980 (Lubrizol), un polymère d'acide acrylique réticulé obtenu par polymérisation par précipitation. Ces produits commerciaux sont typiquement utilisés dans des compositions cosmétiques ou dermatologiques comme agent épaississant,

L'évolution de la viscosité d'une solution comprenant 0,5% en masse de polymère épaississant est étudiée, en fonction de la concentration en EDTA,

Plus précisément, dans un bêcher de 400 ml on introduit 250 g d'eau déionisée, puis sous agitation mécanique (tripâle - 500 tours par minute), on ajoute progressivement la quantité d'émulsion inverse désirée pour obtenir une solution contenant 0,5% en masse de polymère actif. Le pH est ensuite ajusté à 7 +/-0,1 avec de l'hydroxyde de sodium, A ce pH, 100% des fonctions acide présentes sur le polymère sont neutralisées. Une solution aqueuse d'EDTA (5% massique) est ajoutée à la concentration désirée. La solution est laissée 15 minutes sous agitation, puis 5 minutes au repos, La viscosité est alors mesurée à l'aide d'un viscosimètre Brookfield de type RVT avec le module 4 et une vitesse de rotation 20 tours par minute.

Les résultats sont consignés dans le **Tableau 2.**

**Tableau 2** - Mesure de viscosité d'une solution contenant 0,5% en masse de polymère avec ajout d'EDTA

| Ex | NFA (%) | CM (mmol/g) | Viscosité (cps) | | | | |
|---|---|---|---|---|---|---|---|
| | | | 0% EDTA | 0,025% EDTA | 0,05% EDTA | 0,1% EDTA | 0,2% EDTA |
| 1 | 0% | 2,8 | 13500 | 13000 | 12000 | 11000 | 9200 |
| 2 | 0% | 3,2 | 16000 | 14000 | 12000 | 10250 | 7500 |

(suite)

| Ex | NFA (%) | CM (mmol/g) | Viscosité (cps) | | | | |
|---|---|---|---|---|---|---|---|
| | | | 0% EDTA | 0,025% EDTA | 0,05% EDTA | 0,1% EDTA | 0,2% EDTA |
| 3 | 0% | 1,8 | 11500 | 11000 | 10250 | 9750 | 9000 |
| 4 | 3.5% | 2,8 | 14000 | 13000 | 12000 | 11250 | 9500 |
| 5 | 19% | 3,5 | 18000 | 12000 | 7500 | 5000 | 3500 |
| 6 | 0% | 2,8 | 35000 | 32000 | 29500 | 23500 | 14000 |
| Comp.1 | 0% | 0,9 | 5500 | 2500 | 1500 | 950 | 450 |
| Comp.2 | 52% | 3,7 | 20000 | 14000 | 5000 | 800 | <50 |
| Comp.3 | 0% | 3,7 | NA | NA | NA | NA | NA |
| Comp.4 | 50% | 2,8 | 16000 | 12000 | 6000 | 750 | 300 |
| Sepigel® 305 | | | 10000 | 5000 | <50 | <50 | <50 |
| Novemer® EC2 | | | 20800 | 19000 | 16400 | 12700 | 7300 |
| Carbopol® 980 | Non applicable car méthode de polymérisation différente | | 39000 | 32000 | 26000 | 17000 | 8000 |
| NFA : Neutralisation des fonctions acide (%) à l'issue de la polymérisation CM : Concentration en monomère en mmol/g de phase aqueuse | | | | | | | |

**Tableau 3** - Pourcentage de viscosité maintenue avec l'addition d'EDTA

| Le pourcentage de viscosité maintenue correspond au rapport entre la viscosité finale en présence d'EDTA sur la viscosité initiale sans EDTA multiplié par 100. | | | | |
|---|---|---|---|---|
| Ex | 0,025% EDTA | 0,05% EDTA | 0,1% EDTA | 0,2% EDTA |
| 1 | 96% | 89% | 82% | 68% |
| 2 | 88% | 75% | 64% | 47% |
| 3 | 96% | 89% | 85% | 78% |
| 4 | 93% | 86% | 80% | 68% |
| 5 | 67% | 42% | 28% | 19% |
| 6 | 91,4% | 84,3% | 67,1% | 40% |
| Comp.1 | 45% | 27% | 17% | 8% |
| Comp.2 | 70% | 25% | 4% | <0.25% |
| Comp.3 | NA | NA | NA | NA |
| Comp.4 | 75% | 38% | 5% | 2% |
| Sepigel® 305 | 50% | <0,5% | <0,5% | <0,5% |
| Novemer® EC2 | 91% | 79% | 61% | 35% |
| Carbopol® 980 | 82% | 66,7% | 43,6% | 20,5% |

Les polymères des exemples 1 à 6 permettent d'obtenir une très bonne, voire excellente résistance aux électrolytes comparativement aux polymères des exemples comparatifs 1 à 4.

Lorsque l'on compare les exemples 1 et 6, on note que le polymère offrant la meilleure efficacité épaississante sans électroiytes (polymère 6) est un peu moins résistant aux électroiytes que le polymère 1, L'homme de l'art pourra aisément grâce à ses connaissances trouver le meilleur compromis entre l'efficacité épaississante et la résistance aux électroiytes en faisant varier des paramètres de polymérisation.

Le Sepigel® 305 procure un effet épaississant intéressant, mais a une très faible résistance aux électrolytes.

Le Novemer® EC2 permet d'obtenir à la fois un bon épaississement et une résistance aux électrolytes satisfaisante, mais il conduit à une texture plutôt structurée voire gélifiée ne correspondant pas aux attentes du consommateur, comme cela sera explicité dans la partie relative aux exemples de compositions cosmétiques et dermatologiques,

Le Carbopol® 980 a une très bonne efficacité épaississante, comparable à celle du polymère de l'exemple 6. Cependant, sa résistance aux électrolytes est inférieure à celle du polymère de l'exemple 6.

## II. Etude comparative par rapport aux polymères en émulsion inverse proposés dans l'art antérieur pour une application en cosmétique

L'effet épaississant de polymères obtenus par polymérisation en émulsion inverse tels que décrits dans l'art antérieur, et de polymères utilisés dans le cadre de l'invention, toutes autres conditions égales par ailleurs, a été comparé,

Les exemples de différents documents de l'art antérieur ont été reproduits et ensuite, seule(s) la concentration et/ou le pourcentage de neutralisation a(ont) été modifié(s) pour correspondre à l'invention. Ensuite, la mise en oeuvre des émulsions inverses et les mesures de viscosités ont été faites selon le même protocole que celui décrit précédemment.

Dans ce qui suit, AA désigne l'acide acrylique, AM l'acrylamide, ATBS l'acide 2-acrylamido-2-méthylpropane sulfonique.

### a. EP 0 503 853

Les exemples 2 et 7 décrits pages 5-6 du brevet EP 0 503 853 ont été reproduits. Ensuite, ces exemples 1, 2 et 7 ont été modifiés pour correspondre à l'invention, Seule(s) la concentration et/ou le pourcentage de neutralisation a(ont) donc été modifié(s) pour correspondre à l'invention. Ensuite, la mise en oeuvre des émulsions inverses et les mesures de viscosités ont été faites selon le même protocole que celui décrit précédemment.

Dans ce qui suit, AA désigne l'acide acrylique et AM l'acrylamide.

### Exemple 7

L'exemple 7 correspond à l'exemple 2 dans lequel les quantités de monomère acide acrylique, de NaOH et de MBA ont été diminuées et remplacées par de l'eau désionisée de manière à obtenir la même quantité de phase aqueuse, une concentration totale en monomères de 3,4 mmol/g de phase aqueuse, au lieu de 4,3 et une neutralisation des fonctions acide de 15% au lieu de 100%.

### Exemple 8

L'exemple 8 correspond à l'exemple 7 dans lequel les quantités de monomères acrylamide, acide acrylique et de MBA ainsi que de NaOH ont été diminuées et remplacées par de l'eau désionisée de manière à obtenir une concentration totale en monomères de 3,4 mmol/g de phase aqueuse, au lieu de 4,3 et une neutralisation des fonctions acide de 15% au lieu de 100%,

Les résultats sont consignés dans le **Tableau 4.**

**Tableau 4**

| Exemple | Neutralisation des fonctions acide (%) | Monomère(s) ratio (mol%) | [monomère(s)] mmol/g phase aq. | Viscosité 0,16% dans l'eau (cps) |
|---|---|---|---|---|
| Ex2 de EP 0 503 853 | 100% | AA: 100% Avec agent de ramification | 4,3 | 20 |
| 7 | 15% | AA : 100% Avec agent de ramification | 3,4 | 6500 |

(suite)

| Exemple | Neutralisation des fonctions acide (%) | Monomère(s) ratio (mol%) | [monomère(s)] mmol/g phase aq. | Viscosité 0,16% dans l'eau (cps) |
|---|---|---|---|---|
| Ex7 de EP 0 503 853 | 100% | AM: 50% AA: 50% Avec agent de ramification | 4,3 | 50 |
| 8 | 15% | AM: 50% AA: 50% Avec agent de ramification | 3,4 | 4500 |

L'association des deux caractéristiques essentielles pour préparer les polymères utilisés dans le cadre de l'invention, à savoir une faible concentration en monomères dans la phase aqueuse associée à un faible taux de neutralisation des monomères à fonctions acide permet d'obtenir des polymères procurant un effet épaississant grandement amélioré.

La résistance aux électrolytes des polymères de l'art antérieur « peu » viscosants (seulement 20 à 50 cps à 0,16%) n'a pas été testée car ces polymères présentent peu d'intérêt par rapport à leurs équivalents préparés dans les conditions utilisées dans le cadre de l'invention. De plus, le Seplgel® 305 de la société SEPPIC correspond à l'Invention définie dans le brevet EP 0 503 853 et la partie précédente montre que ce polymère a une faible résistance aux électrolytes.

**b. WO 2005/097834**

L'exemple 2 décrit page 14 de la demande de brevet WO 2005/097834 a été reproduit. Ensuite, le pourcentage de neutralisation et la concentration en monomères ont été abaissés pour correspondre à invention (exemple 9).

Les résultats sont consignés dans le **Tableau 5.**

**Tableau 5**

| Exemple | Neutralisation | Monomère ratio (mol%) | [monomère] mmol/g phase aq. | Viscosité 0,16% dans l'eau (cps) |
|---|---|---|---|---|
| Ex 2 de WO2005/0978 34 | 37,5% | AA: 100% | 4,7 | 20 |
| Exemple 9 | 18% | AA: 100% | 3,4 | 2500 |

Encore, une fois, ces essais montrent intérêt d'utiliser des polymères préparés selon l'invention par rapport aux procédés de l'art antérieur, puisqu'il permet de considérablement améliorer le pouvoir épaississant des polymères obtenus.

**III - Efficacité et aspect dans des compositions cosmétiques et dermatologiques**

Les tests suivants montrent l'intérêt d'utiliser les polymères obtenus dans les conditions de concentration et % de neutralisation, définies dans le cadre de l'invention dans des compositions cosmétiques ou dermatologiques. De tels polymères apportent non seulement un bon épaississement des compositions, y compris en présence d'électrolytes, mais également leur procure un aspect attrayant au toucher et à l'application.

Des crèmes, lotions, gels cosmétiques ou dermatologiques contenant des électrolytes sont formulés avec les émulsions inverses des exemples 1 et 6 et le Novemer® EC2.

Pour chacune des compositions numérotées de 1 à 3 ci-dessous, le protocole de préparation appliqué est le suivant :

Préparation de la partie A :

- Dans un bécher de 400 ml on ajoute l'eau délonisée
- Sous agitation d'une pâle défloculeuse à 500 tours par minute, on ajoute l'émulsion inverse

- On ajuste le pH à la fourchette désirée à l'aide d'hydroxyde de sodium ou de l'acide citrique, respectivement mis en solution aqueuse à 10% et 50%
- Les autres ingrédients sont ajoutés
- 15 minutes d'agitation suffisent à obtenir une pâte visqueuse homogène

Préparation de la partie B :

- Dans un bécher de 400 ml on mélange les ingrédients
- 5 minutes sous agitation d'une tripâle à 250 tours par minutes suffisent pour assurer l'homogénéité du mélange

A température ambiante, on transfert progressivement la partie B dans la partie A. L'agitation est alors portée à 2000 tours par minutes durant 10 minutes.

La formulation est alors laissée au repos pendant 60 minutes avant de contrôler la viscosité à l'aide d'un Viscosimètre RVT, 20 tours/minute, module 6.

**Composition N°1 :** Base pour gel crème

Partie A :

| Ingrédients (100 parts) | Exemple 1 | Novemer® EC 2 | Exemple 6 |
|---|---|---|---|
| Eau déionisée | 75,0 | 75,0 | 75,0 |
| EDTA (solution aqueuse à 5% en masse) | 2,0 | 2,0 | 2,0 |
| Polymère* | 2,1 (1) | 7,5 (1,5) | 0,9 (0,42) |
| Hydroxyde de sodium | 1,3 | - | 0,65 |
| Acide citrique | - | 1,4 | - |

Partie B :

| Eau déionisée | 17,25 | 11,75 | 19,1 |
|---|---|---|---|
| Glycérine @ 85% | 2,35 | 2,35 | 2,35 |

Résultats :

| pH | 5,4 | 5,4 | 5,5 |
|---|---|---|---|
| Viscosité finale en cps | 21500 | 22500 | 20200 |

*Polymère sous forme d'émulsion inverse ou commerciale dans le cas du Novemer® EC2. Le pourcentage de polymère dans l'émulsion inverse de l'exemple 1 est de 47,6%, dans l'émulsion inverse de l'exemple 6 de 46,4% et dans le Novemer® EC2 de 20%. Entre parenthèse est noté le pourcentage de polymère en masse dans la composition.

Les polymères utilisés dans le cadre de l'invention permettent de viscosifier efficacement la base pour gel crème même si celui-ci contient une quantité de 0,1% d'EDTA, Ils permettent une moindre consommation de polymère pour épaissir la base pour gel crème à une viscosité standard d'environ 20 000 cps : 1% de polymère de l'exemple 1 ou 0.42% de polymère de l'exemple 6 au lieu de 1,5% de Novemer® EC2, soit respectivement une baisse de dosage de 33% et de 72%, ce qui est très significatif.

De plus la base pour gel crème obtenue avec les polymères des exemples 1 et 6 présentent un aspect très attrayant, Les sensations de son application sur la peau sont douces et agréables contrairement à la base obtenue avec le Novemer® EC2 qui, plus structurée, donne une sensation plutôt désagréable.

Un essai complémentaire est conduit en utilisant les polymères 1 et 6 sous forme de poudre, à la place de la version en émulsion inverse. Plus précisément les polymères 1 et 6 sous forme de poudre, obtenus à partir des émulsions inverses, sont mis en solution dans de l'eau, ladite solution étant ajoutée dans la composition base pour gel crème à épaissir. Les résultats de viscosité et de texture sont sensiblement les mêmes que ceux obtenus avec les émulsions inverses.

**Composition N°2** : Lotion à base de silicone

Partie A :

| Ingrédients (100 parts) | Exemple 1 | Novemer® EC 2 | Exemple 6 |
|---|---|---|---|
| Eau déionisée | 76,6 | 70,2 | 77,2 |
| EDTA (solution aqueuse à 5% en masse) | 2 | 2 | 2 |
| Polymère* | 3,3 (1.57) | 12,5 (2,5) | 3,4 (1,58) |
| Hydroxyde de sodium | 3,1 | - | 2,35 |
| Acide citrique | - | 0,3 | - |

Partie B :

| Dimethicone | 10 | 10 | 10 |
|---|---|---|---|
| Palmitate d'octyle | 5 | 5 | 5 |

Résultats :

| pH | 5,9 | 6,0 | 5,9 |
|---|---|---|---|
| Viscosité finale en cps | 11600 | 9000 | 11200 |

Les polymères utilisés dans le cadre de l'invention permettent de viscosifier efficacement la lotion à base de silicone même si celle-ci contient une quantité de 0.1% d'EDTA. Ils permettent une moindre consommation de polymère pour épaissir la lotion à une viscosité standard d'environ 10.000 cps : 1.57% de polymère de l'exemple 1 ou 1.58% de polymère de l'exemple 6 au lieu de 2.5% de Novemer® EC2, soit une baisse de dosage de 37%, ce qui est très significatif.

De plus, la lotion obtenue avec les polymères des exemples 1 et 6 présentent un aspect très attrayant. Les sensations de son application sur la peau sont douces et agréables contrairement à la base obtenue avec le Novemer® EC2 qui, plus structurée, donne une sensation plutôt désagréable.

**Composition N°3** : Base crème capillaire

Partie A :

| Ingrédients (100 parts) | Exemple 1 | Novemer® EC 2 | Exemple 6 |
|---|---|---|---|
| Eau délonisée | 92,4 | 90,2 | 92,75 |
| EDTA (solution aqueuse à 5% en masse) | 2 | 2 | 2 |
| Polymère* | 1,0 (0.48) | 3,5 (0,7) | 0,75 (0,35) |
| Hydroxyde de sodium | 0,6 | - | 0,5 |
| Acide citrique | - | 0,3 | - |

Partie B :

| Huile d'olive | 4 | 4 | 4 |
|---|---|---|---|

Résultats :

| pH | 5,5 | 5,6 | 5,5 |
|---|---|---|---|
| Viscosité finale en cps | 12000 | 13300 | 11500 |

Les polymères utilisés dans le cadre de l'invention permettent de viscosifier efficacement la crème même si celle-ci contient une quantité de 0.1% dBDTA. Ils permettent une moindre consommation de polymère pour épaissir la crème à une viscosité standard d'environ 12.000 cps : 0.48% de polymère de l'exemple 1 ou 0.35% de polymère de l'exemple 6 au lieu de 0.7% de Novemer® EC2, soit respectivement une baisse de dosage de 31% et de 50%, ce qui est très significatif.

De plus, la crème obtenue avec les polymères des exemples 1 et 6 présentent un aspect très attrayant. Les sensations de son application sur la peau sont douces et agréables contrairement à la base obtenue avec le Novemer® EC2 qui, plus structurée, donne une sensation plutôt désagréable.

## Composition N°4 : Shampoing

La procédure consiste à d'abord disperser le polymère dans l'eau déionisée sous agitation d'une tripâle à 300 tour / min. Le propylène glycol, et le tensioactif sont ensuite ajoutés. Après ajustement du pH la bétaïne est ajoutée lentement sous agitation. La formulation finale est ensuite agitée 30 minutes.

| Ingrédients (100 parts) | Exemple 1 | Carbopol® 980 |
|---|---|---|
| Eau délonisée | 57,9 | 58,9 |
| Polymère | 2,0 | 1,0 |
| Propylèneglycol | 6,0 | 6,0 |
| Sodium Laureth Sulfate 3EO (28%) | 30,0 | 30,0 |
| Disodium EDTA | 0,1 | 0,1 |
| NaOH | QSP pH = 6,7 +/ 0,2 | |
| Cocamidopropylbétaïne | 4,0 | 4,0 |

Résultats

| pH | 6,7 | 6,8 |
|---|---|---|
| Viscosité LVT, 30 rpm | 6500 cps | 2500 cps |

Le polymère utilisé dans le cadre de l'invention permet de viscosifier efficacement une formulation contenant des tensio-actifs détergents. La formulation est stable et présente d'excellentes caractéristiques d'écoulement

## Composition N°5 : Base pour gel douche

La procédure est la même que pour l'exemple 4 avec la composition suivante :

| Ingrédients (100 parts) | Exemple 1 | Carbopol® 980 |
|---|---|---|
| Eau délonisée | | |
| Polymère | 2,0 | 1,0 |
| Sodium Laureth Sulfate 2EO (28%) | 32,0 | 32,0 |
| NaOH | QSP pH = 5,5 +/ 0,2 | |
| Cocamidopropylbétaïne | 7,0 | 4,0 |
| Glycérine | 1,5 | 1,5 |

(suite)

| Ingrédients (100 parts) | Exemple 1 | Carbopol® 980 |
|---|---|---|
| Polyquaternium 7 | 1,0 | 1,0 |

Résultats

| | | |
|---|---|---|
| pH | 5,4 | 5,5 |
| Viscosité LVT, 30 rpm | 3500 cps | 1500 cps |

Le polymère utilisé dans le cadre de l'invention permet d'obtenir une formulation compatible avec tous les autres ingrédients (en particulier avec les bétaïnes et les conditionneurs) dans une plage de viscosité facilitant l'application.

**Revendications**

1. Utilisation, pour la fabrication d'une composition cosmétique ou dermatologique comprenant au moins une phase aqueuse, d'un polymère branché ou réticulé composé de la répétition d'une ou plusieurs unités monomériques, avec au moins une des unités monomériques qui correspond à un monomère comportant un groupe acrylique et au moins 30% molaire des unités monomériques qui sont porteuses d'au moins une fonction acide faible éventuellement sous forme neutralisée, ledit polymère étant obtenu :
   par polymérisation d'une solution aqueuse d'un ou plusieurs monomères en émulsion inverse eau dans huile, au moins un des monomères utilisés étant un monomère acrylique et un ou plusieurs des monomères utilisés étant un monomère porteur d'au moins une fonction acide faible, le pourcentage molaire de monomères porteurs d'au moins une fonction acide faible par rapport à l'ensemble des monomères utilisés étant au moins de 30%, la phase aqueuse contenant au moins un monomère jouant le rôle d'agent de ramification, de sorte que la polymérisation conduit à un polymère branché ou réticulé, **caractérisé en ce que** :

   i) la polymérisation est réalisée avec une concentration de la totalité des monomères en solution aqueuse appartenant à la gamme allant de 1,3 mmol à 3,6 mmol par gramme de solution aqueuse,
   ii) pendant la polymérisation, au plus 20% des fonctions acide présentes sur les monomères possédant au moins une fonction acide se trouvent sous forme neutralisée.

2. Utilisation, pour épaissir une composition cosmétique ou dermatologique comprenant au moins une phase aqueuse, d'un polymère branché ou réticulé composé de la répétition d'une ou plusieurs unités monomériques, avec au moins une des unités monomériques qui correspond à un monomère comportant un groupe acrylique et au moins 30% molaire des unités monomériques qui sont porteuses d'au moins une fonction acide faible éventuellement sous forme neutralisée, ledit polymère étant obtenu :
   par polymérisation d'une solution aqueuse d'un ou plusieurs monomères en émulsion inverse eau dans huile, au moins un des monomères utilisés étant un monomère acrylique et un ou plusieurs des monomères utilisés étant un monomère porteur d'au moins une fonction acide faible, le pourcentage molaire de monomères porteurs d'au moins une fonction acide faible par rapport à l'ensemble des monomères utilisés étant au moins de 30%, la phase aqueuse contenant au moins un monomère jouant le rôle d'agent de ramification, de sorte que la polymérisation conduit à un polymère branché ou réticulé, **caractérisé en ce que** :

   la polymérisation est réalisée avec une concentration de la totalité des monomères en solution aqueuse appartenant à la gamme allant de 1,3 mmol à 3,6 mmol par gramme de solution aqueuse,
   ii) pendant la polymérisation, au plus 20% des fonctions acide présentes sur les monomères possédant au moins une fonction acide se trouvent sous forme neutralisée;

3. Utilisation selon la revendication 1 ou 2 **caractérisée en ce que** le polymère comporte un pourcentage de fonctions acide neutralisées de 30 à 100% par rapport à la totalité des fonctions acide présentes sur le polymère, obtenu par une étape de neutralisation au moins partielle des fonctions acides présentes sur le polymère réalisée après la polymérisation, mais avant ou après la préparation de la composition.

**EP 3 055 031 B1**

4. Composition cosmétique ou dermatologique comprenant au moins une phase aqueuse et un polymère branché ou réticulé composé de la répétition d'une ou plusieurs unités monomériques, avec au moins une des unités monomériques qui correspond à un monomère comportant un groupe acrylique et au moins 30% molaire des unités monomériques qui sont porteuses d'au moins une fonction acide faible au moins partiellement sous forme neutralisée, le pourcentage de fonctions acide neutralisées par rapport à la totalité des fonctions acide présentes sur le polymère étant de 30 à 100%, ledit polymère étant obtenu :

  par polymérisation d'une solution aqueuse d'un ou plusieurs monomères en émulsion inverse eau dans huile, au moins un des monomères utilisés étant un monomère acrylique et un ou plusieurs des monomères utilisés étant un monomère porteur d'au moins une fonction acide faible, le pourcentage molaire de monomères porteurs d'au moins une fonction acide faible par rapport à l'ensemble des monomères utilisés étant au moins de 30%, la phase aqueuse contenant au moins un monomère jouant le rôle d'agent de ramification, de sorte que la polymérisation conduit à un polymère branché ou réticulé, **caractérisé en ce que** :

    la polymérisation est réalisée avec une concentration de la totalité des monomères en solution aqueuse appartenant à la gamme allant de 1,3 mmol à 3,6 mmol par gramme de solution aqueuse,
    ii) pendant la polymérisation, au plus 20% des fonctions acide présentes sur les monomères possédant au moins une fonction acide se trouvent sous forme neutralisée ;

  la polymérisation étant suivie d'une étape de neutralisation au moins partielle des fonctions acides présentes réalisée avant ou après l'incorporation du polymère dans la composition.

5. - Utilisation selon la revendication 1, 2 ou 3 ou composition selon la revendication 4 **caractérisée en ce que**, pendant la polymérisation, au plus 10%, de préférence au plus 5%, et préférentiellement au plus 2%, des fonctions acide présentes sur les monomères possédant au moins une fonction acide se trouvent sous forme neutralisée.

6. Utilisation selon l'une des revendications 1 à 3 ou 5 ou composition selon la revendication 4 ou 5 **caractérisée en ce que** toutes les fonctions acide présentes sur les monomères se trouvent sous forme acide libre, pendant la polymérisation.

7. Utilisation selon l'une des revendications 1 à 3, 5 ou 6 ou composition selon l'une des revendications 4 à 6 **caractérisée en ce que** la polymérisation est réalisée avec une concentration de la totalité des monomères en solution aqueuse appartenant à la gamme allant de 1,7 à 3,3 mmol par gramme de solution aqueuse.

8. Utilisation selon l'une des revendications 1 à 3 ou 5 à 7 ou composition selon l'une des revendications 4 à 7 **caractérisée en ce que** le polymère comporte un pourcentage molaire d'unités monomériques porteuses d'une ou plusieurs fonction(s) acide faible, par rapport à l'ensemble des unités monomériques porteuses d'une fonction acide, d'au moins 50 %, préférentiellement d'au moins 70%, très préférentiellement d'au moins 80%.

9. Utilisation selon l'une des revendications 1 à 3 ou 5 à 8 ou composition selon l'une des revendications 4 à 8 **caractérisée en ce que** tous les monomères utilisés pour la préparation du polymère sont des monomères possédant au moins une insaturation éthylénique.

10. Utilisation selon l'une des revendications 1 à 3 ou 5 à 9 ou composition selon l'une des revendications 4 à 9 **caractérisée en ce que** la ou les unité(s) monoménque(s) porteuse(s) d'au moins une fonction acide faible, sous forme libre, est(sont) choisie(s) parmi l'acide acrylique, l'acide méthacrylique, l'acide itaconique, l'acide crotonique, l'acide maléique et l'acide fumarique, l'acide acrylique étant préféré.

11. Utilisation selon l'une des revendications 1 à 3 ou 5 à 10 ou composition selon l'une des revendications 4 à 10 **caractérisée en ce que** le polymère est un copolymère comportant au moins une unité monomérique neutre choisie parmi l'acrylamide, le méthacrylamide, le N,N-diméthylacrylamide, le N-vinylméthylacétamide, le N-vinylformamlde, l'acétate de vinyle, le diacétoneacrylamide, le N-isopropyl acrylamide, le N-[2-hydroxy-1,1-bis(hydroxyméthyl)éthyl]propénamide, l'acrylate de (2-hydroxyéthyle), l'acrylate de (2,3-dihydroxypropyle), le méthacrylate de méthyle, le méthacrylate de (2-hydroxyéthyle), le méthacrylate de (2,3-dihydroxy propyle) et la vinylpyrrolidone.

12. Utilisation selon l'une des revendications 1 à 3 ou 5 à 11 ou composition selon l'une des revendications 4 à 11 **caractérisée en ce que** toutes les unités monomériques porteuses d'au moins une fonction acide présentes dans le polymère sont des unités monomériques porteuses d'une ou plusieurs fonction(s) acide faible.

**13.** Utilisation ou composition selon la revendication 12 **caractérisée en ce que** le polymère présent dans la composition est un copolymère acide acrylique/acrylamide avec de 30 à 100% des fonctions acide acrylique sous forme neutralisée.

**14.** Utilisation selon l'une des revendications 1 à 3 ou 5 à 11 ou composition selon l'une des revendications 4 à 11 **caractérisée en ce que** le polymère est un copolymère comportant au moins une unité monomérique porteuse d'une ou plusieurs fonctlon(s) acide fort.

**15.** Utilisation ou composition selon la revendication 14 **caractérisée en ce que** le pourcentage molaire en unités monomériques porteuses d'une ou plusieurs fonction(s) acide fort par rapport à l'ensemble des unités monomériques est inférieur à 50%, et préférentiellement inférieur à 30%.

**16.** Utilisation ou composition selon la revendication 14 ou 15 **caractérisée en ce que** la ou les unité(s) monomérique(s) porteuse(s) d'une ou plusieurs fonction(s) acide fort, sous forme libre, est(sont) choisie(s) parmi les acides acrylamidoalkylsulfonique tel que l'acide 2-acrylamido-2-rnéthylpropane sulfonique,

**17.** Utilisation ou composition selon la revendication 14, 15 ou 16 **caractérisée en ce que** le polymère présent dans la composition est un copolymère acide 2-acrylamido-2-méthylpropane sulfonique/acide acrylique ou acide 2-acrylamido-2-méthylpropane sulfonique/acide acrylique/acrylamide, avec de 30 à 100% des fonctions acide présentes sur le polymère qui sont sous forme neutralisée,

**18.** Utilisation selon l'une des revendications 1 à 3 ou 5 à 17 ou composition selon l'une des revendications 4 à 17 **caractérisée en ce que** l'agent de ramification est choisi parmi le méthylènebisacrylamide (MBA), l'éthylène glycol di-acrylate, le polyéthylène glycol diméthacrylate, le diacrylamide, le cyanométhylacrylate, le vinyloxyéthylacrylate, le vinyloxyméthacrylate, la triallylamine, le formaldéhyde, le glyoxal, les glycidyléthers comme l'éthylèneglycol diglycidyléther, les époxy et leur mélange.

**19.** Utilisation selon l'une des revendications 1 à 3 ou 5 à 18 ou composition selon l'une des revendications 4 à 18 **caractérisée en ce que** la quantité d'agent de ramification est comprise entre 5 et 10 000 ppm en masse par rapport à la masse totale de monomères, et préférentiellement entre 100 et 5 000 ppm.

**20.** Utilisation selon l'une des revendications 1 à 3 ou 5 à 19 ou composition selon l'une des revendications 4 à 19 **caractérisée en ce que** la réaction de polymérisation est menée en présence d'un agent émulsionnant eau dans huile.

**21.** Utilisation selon l'une des revendications 1 à 3 ou 5 à 20 ou composition selon l'une des revendications 4 à 20 **caractérisée en ce que** la polymérisation est menée avec un agent de transfert choisi parmi le méthanol, l'alcool isopropylique, l'hypopnosphite de sodium, le 2-mercaptoéthanol, le méthallysulfonate de sodium et leur mélange.

**22.** Utilisation ou composition selon la revendication 21 **caractérisée en ce que** la quantité d'agent de transfert est comprise entre 0 et 5000 ppm en masse par rapport à la masse totale de monomères, et préférentiellement entre 10 et 2500 ppm.

**23.** Utilisation selon l'une des revendications 1 à 3 ou 5 à 22 ou composition selon l'une des revendications 4 à 22 **caractérisée en ce que** la polymérisation est suivie d'une ou plusieurs des étapes suivantes :

- une dilution ou une concentration de rémulsion obtenue,
- une isolation pour obtenir le polymère sous la forme d'une poudre.

**24.** Utilisation selon l'une des revendications 1 à 3 ou 5 à 23 ou composition selon l'une des revendications 4 à 23 **caractérisée en ce que** le polymère branché ou réticulé introduit dans la composition a une viscosité, à 0,16% en masse dans de l'eau déminéralisée dont le pH est ajusté à 7 +/-0,1 avec de l'hydroxyde de sodium, mesurée à 25°C avec un appareil Brookfiled de type RVT (vitesse de rotation 20 t/min), appartenant à la gamme allant de 2000 mPa.s à 100 000 mPa.s, notamment à la gamme allant de 3000 mPa.s à 50 000 mPa.s.

**25.** Utilisation selon l'une des revendications 1 à 3 ou 5 à 24 ou composition selon l'une des revendications 4 à 24 **caractérisée en ce que** la composition est adaptée pour une application topique.

26. Utilisation selon l'une des revendications 1 à 3 ou 5 à 25 ou composition selon l'une des revendications 4 à 25 **caractérisée en ce que** la composition se présente sous la forme d'un lait, d'une lotion, d'un gel, d'une crème, d'un gel crème, d'un savon, d'un bain moussant, d'un baume, d'un shampooing ou d'un après-shampooing.

27. Utilisation selon l'une des revendications 1 à 3 ou 5 à 26 ou composition selon l'une des revendications 4 à 26 **caractérisée en ce que** la composition comprend un électrolyte, de préférence, choisi parmi les extraits végétaux contenant des ions monovalents ou divalents comme les acides de fruits, les principes actifs tels que les hydroxy acides pour leur effet anti-âge, les agents hydratants tels que l'acide carboxylique pyrrolidone, des agents chelatants comme l'acide éthylène diamine tétraacétique (EDTA), les filtres UV comme l'acide sulfonique phénylbenzlmidazole, certains conservateurs ou encore des sels comme le sel d'alun.

28. Utilisation selon l'une des revendications 1 à 3 ou 5 à 27 ou composition selon l'une des revendications 4 à 27 **caractérisée en ce que** la composition comprend de 0,01% à 10% en masse de polymère branché ou réticulé, par rapport à la masse totale de la composition, et préférentiellement de 0,1 à 5% en masse de polymère branché ou réticulé.

29. Utilisation selon l'une des revendications 1 à 3 ou 5 à 28 ou composition selon l'une des revendications 4 à 28 **caractérisée en ce que** la composition comprend au moins un agent actif choisi parmi choisi parmi les agents hydratants, les agents bronzants, les filtres solaires, les vitamines, les oligo-éléments, les agents anti-rides ou anti-âge, les extraits botaniques, les agents à visée amincissante, les agents anti-radicalaires, les agents anti-chutes des cheveux, les agents antipelliculaires, les tensioactifs nettoyants, les polymères de conditionnement de la peau, les émollents et les principes actifs pharmaceutiques tels que les agents anti-fongiques, les agents anti-bactériens, les agents anti-inflammatoires, les myorelaxants, les antibiotiques, les agents antiviraux, les analgésiques, les anti-bistaminiques, les agents antipruritiques, les agents antipyrétiques, les agents anesthésiques, les agents diagnostiques, les hormones, les rehausseurs de croissance cutanée, les modulateurs pigmentaires, les agents antiprolifératifs, les agents antipsoriatiques, les rétinoïdes, les médicaments anti-acné, les agents antinéopastiques, les agents photothérapeutiques, les agents kératolytiques, et leurs analogues.

30. Utilisation selon l'une des revendications 1 à 3 ou 5 à 29 ou composition selon l'une des revendications 4 à 29 **caractérisée en ce que** la composition comprend au moins un additif, et notamment au moins un auxiliaire de formulation, par exemple choisi parmi les agents chélatants, les agents de neutralisation et d'ajustement du PH, les opacifiants, les conservateurs, les agents d'étalement, les émollients, les polymères filmogènes, les antioxydants, les parfums, les agents réfléchissants, les agents coalescents et les mélanges de ceux-ci.

31. Utilisation selon l'une des revendications 1 à 3 ou 5 à 30 ou composition selon l'une des revendications 4 à 30 **caractérisée en ce que** la composition est une émulsion d'une phase huileuse dans une phase aqueuse ou une émulsion d'une phase aqueuse dans une phase huileuse.

32. Utilisation ou composition selon la revendication 31 **caractérisée en ce que** la phase huileuse est constituée d'une huile végétale ou de plante, d'une huile silicone, d'une huile hydrocarbonée fluorée, d'une huile hydrocarbonée, d'une huile minérale, de polyisobutène, d'isohexadécane, d'un triglycéride caprylique/caprique, d'octanoate de cétéaryle, de benzoate d'alkyle en $C_{12}$-$C_{14}$, ou d'un de leur mélange.

33. Utilisation selon l'une des revendications 1 à 3 ou 5 à 32 ou composition selon l'une des revendications 4 à 32 **caractérisée en ce que** la composition comprend un agent émulsionnant eau dans huile et/ou un agent émulsionnant huile dans eau.

34. Utilisation selon l'une des revendications 1 à 3 ou 5 à 33 ou composition selon l'une des revendications 4 à 33 **caractérisée en ce que** le polymère branché ou réticulé est hydrosoluble ou hydro-gonflant.

35. Utilisation d'une composition selon l'une des revendications 4 à 34 pour le traitement cosmétique des matières kératiniques telles que la peau, le cuir chevelu, les cils, les sourcils, les ongles, les cheveux et/ou les muqueuses, à l'exclusion de tout traitement thérapeutique.

36. Utilisation selon la revendication 35 qui comprend l'application de la composition sur les matières kératiniques, éventuellement suivie d'un rinçage à l'eau.

**Patentansprüche**

1. Verwendung, zur Herstellung einer kosmetischen oder dermatologischen Zusammensetzung, die mindestens eine wässrige Phase umfasst, eines verzweigten oder vernetzten Polymers, das aus der Wiederholung von einer oder mehreren monomeren Einheiten besteht, wobei mindestens eine der monomeren Einheiten einem Monomer entspricht, das eine Acrylgruppe umfasst, und mindestens 30 Mol-% der monomeren Einheiten mindestens eine schwache Säurefunktion, gegebenenfalls in neutralisierter Form, tragen, wobei das Polymer erhalten wird: durch Polymerisation einer wässrigen Lösung eines oder mehrerer Monomere in Wasser-in-Öl-Umkehremulsion, wobei mindestens eines der verwendeten Monomere ein Acrylmonomer und eines oder mehrere der verwendeten Monomere ein Monomer ist, das mindestens eine schwache Säurefunktion trägt, wobei der molare Anteil der Monomere, die mindestens eine schwache Säurefunktion tragen, im Verhältnis zur Gesamtheit der verwendeten Monomere mindestens 30 % beträgt, wobei die wässrige Phase mindestens ein Monomer als Verzweigungsmittel enthält, so dass die Polymerisation zu einem verzweigten oder vernetzten Polymer führt, **dadurch gekennzeichnet, dass:**

   i) die Polymerisation mit einer Konzentration aller Monomere in wässriger Lösung im Bereich von 1,3 mmol bis 3,6 mmol pro Gramm wässriger Lösung durchgeführt wird,
   ii) während der Polymerisation bis zu 20 % der auf den Monomeren vorhandenen Säurefunktionen mit mindestens einer Säurefunktion in neutralisierter Form vorliegen.

2. Verwendung, zum Verdicken einer kosmetischen oder dermatologischen Zusammensetzung, die mindestens eine wässrige Phase umfasst, eines verzweigten oder vernetzten Polymers, das aus der Wiederholung von einer oder mehreren monomeren Einheiten besteht, wobei mindestens eine der monomeren Einheiten einem Monomer entspricht, das eine Acrylgruppe umfasst, und mindestens 30 Mol-% der monomeren Einheiten mindestens eine schwache Säurefunktion, gegebenenfalls in neutralisierter Form, tragen, wobei das Polymer erhalten wird: durch Polymerisation einer wässrigen Lösung eines oder mehrerer Monomere in Wasser-in-Öl-Umkehremulsion, wobei mindestens eines der verwendeten Monomere ein Acrylmonomer und eines oder mehrere der verwendeten Monomere ein Monomer ist, das mindestens eine schwache Säurefunktion trägt, wobei der molare Anteil der Monomere, die mindestens eine schwache Säurefunktion tragen, im Verhältnis zur Gesamtheit der verwendeten Monomere mindestens 30 % beträgt, wobei die wässrige Phase mindestens ein Monomer als Verzweigungsmittel enthält, so dass die Polymerisation zu einem verzweigten oder vernetzten Polymer führt, **dadurch gekennzeichnet, dass:**

   i) die Polymerisation mit einer Konzentration aller Monomere in wässriger Lösung im Bereich von 1,3 mmol bis 3,6 mmol pro Gramm wässriger Lösung durchgeführt wird,
   ii) während der Polymerisation bis zu 20 % der auf den Monomeren vorhandenen Säurefunktionen mit mindestens einer Säurefunktion in neutralisierter Form vorliegen;

3. Verwendung nach Anspruch 1 oder 2, **dadurch gekennzeichnet, dass** das Polymer einen Prozentsatz an neutralisierten Säurefunktionen von 30 bis 100 % in Bezug auf alle an dem Polymer vorhandenen Säurefunktionen aufweist, erhalten durch einen zumindest teilweisen Neutralisierungsschritt der auf dem Polymer vorhandenen Säurefunktionen, der nach der Polymerisation, aber vor oder nach der Herstellung der Zusammensetzung durchgeführt wird.

4. Kosmetische oder dermatologische Zusammensetzung, umfassend mindestens eine wässrige Phase und ein verzweigtes oder vernetztes Polymer, das aus der Wiederholung einer oder mehrerer monomerer Einheiten besteht, wobei mindestens eine der monomeren Einheiten einem Monomer mit einer Acrylgruppe entspricht, und mindestens 30 Mol-% der monomeren Einheiten mindestens eine schwache Säurefunktion zumindest teilweise in neutralisierter Form tragen, wobei der Prozentsatz der neutralisierten Säurefunktionen in Bezug auf die Gesamtheit der auf dem Polymer vorhandenen Säurefunktionen 30 bis 100 % beträgt, wobei das Polymer erhalten wird:

   durch Polymerisation einer wässrigen Lösung eines oder mehrerer Monomere in Wasser-in-Öl-Umkehremulsion, wobei mindestens eines der verwendeten Monomere ein Acrylmonomer und eines oder mehrere der verwendeten Monomere ein Monomer ist, das mindestens eine schwache Säurefunktion trägt, wobei der molare Anteil der Monomere, die mindestens eine schwache Säurefunktion tragen, im Verhältnis zur Gesamtheit der verwendeten Monomere mindestens 30 % beträgt, wobei die wässrige Phase mindestens ein Monomer als Verzweigungsmittel enthält, so dass die Polymerisation zu einem verzweigten oder vernetzten Polymer führt, **dadurch gekennzeichnet, dass:**

i) die Polymerisation mit einer Konzentration aller Monomere in wässriger Lösung im Bereich von 1,3 mmol bis 3,6 mmol pro Gramm wässriger Lösung durchgeführt wird,

ii) während der Polymerisation bis zu 20 % der auf den Monomeren vorhandenen Säurefunktionen mit mindestens einer Säurefunktion in neutralisierter Form vorliegen;

wobei auf die Polymerisation ein Schritt der mindestens teilweisen Neutralisierung der vorhandenen Säurefunktionen folgt, der vor oder nach dem Einbringen des Polymers in die Zusammensetzung durchgeführt wird,

5. Verwendung nach Anspruch 1, 2 oder 3 oder Zusammensetzung nach Anspruch 4, **dadurch gekennzeichnet, dass,** während der Polymerisation bis zu 10 %, vorzugsweise bis zu 5 % und vorzugsweise bis zu 2 % der auf den Monomeren vorhandenen Säurefunktionen mit mindestens einer Säurefunktion in neutralisierter Form vorliegen.

6. Verwendung nach einem der Ansprüche 1 bis 3 oder 5 oder Zusammensetzung nach Anspruch 4 oder 5, **dadurch gekennzeichnet, dass** alle auf den Monomeren vorhandenen Säurefunktionen während der Polymerisation in freier Säureform vorliegen.

7. Verwendung nach einem der Ansprüche 1 bis 3, 5 oder 6 oder Zusammensetzung nach einem der Ansprüche 4 bis 6, **dadurch gekennzeichnet, dass** die Polymerisation mit einer Konzentration aller Monomere in wässriger Lösung im Bereich von 1,7 bis 3,3 mmol pro Gramm wässriger Lösung durchgeführt wird.

8. Verwendung nach einem der Ansprüche 1 bis 3 oder 5 bis 7 oder Zusammensetzung nach einem der Ansprüche 4 bis 7, **dadurch gekennzeichnet, dass** das Polymer einen molaren Anteil an Monomereinheiten, die eine oder mehrere schwache Säurefunktion(en) tragen, bezogen auf die Gesamtheit der Monomereinheiten, die eine Säurefunktion tragen, von mindestens 50 %, vorzugsweise mindestens 70 %, sehr bevorzugt mindestens 80 %, umfasst.

9. Verwendung nach einem der Ansprüche 1 bis 3 oder 5 bis 8 oder Zusammensetzung nach einem der Ansprüche 4 bis 8, **dadurch gekennzeichnet, dass** alle Monomere, die für die Herstellung des Polymers verwendet werden, Monomere mit mindestens einer ethylenischen Ungesättigtheit sind.

10. Verwendung nach einem der Ansprüche 1 bis 3 oder 5 bis 9 oder Zusammensetzung nach einem der Ansprüche 4 bis 9, **dadurch gekennzeichnet, dass** die Monomereinheit(en), die mindestens eine schwache Säurefunktion in freier Form tragen, ausgewählt ist (sind) aus Acrylsäure, Methacrylsäure, Itaconsäure, Crotonsäure, Maleinsäure und Fumarsäure, wobei Acrylsäure bevorzugt wird.

11. Verwendung nach einem der Ansprüche 1 bis 3 oder 5 bis 10 oder Zusammensetzung nach einem der Ansprüche 4 bis 10, **dadurch gekennzeichnet, dass** das Polymer ein Copolymer ist, das mindestens eine neutrale monomere Einheit umfasst, ausgewählt aus Acrylamid, Methacrylamid, N,N-Dimethylacrylamid, N-Vinylmethylacetamid, N-Vinylformamid, Vinylacetat, Diacetonacrylamid, N-Isopropylacrylamid, N-[2-Hydroxy-1,1-bis(hydroxymethyl)ethyl]propenamid, (2-Hydroxyethyl)acrylat, (2,3-Dihydroxypropyl)acrylat, Methylmethacrylat, (2-Hydroxyethyl)methacrylat, (2,3-Dihydroxypropyl)methacrylat und Vinylpyrrolidon.

12. Verwendung nach einem der Ansprüche 1 bis 3 oder 5 bis 11 oder Zusammensetzung nach einem der Ansprüche 4 bis 11, **dadurch gekennzeichnet, dass** alle in dem Polymer vorhandenen monomeren Einheiten, die mindestens eine Säurefunktion tragen, monomere Einheiten sind, die eine oder mehrere schwache Säurefunktion(en) tragen.

13. Verwendung oder Zusammensetzung nach Anspruch 12, **dadurch gekennzeichnet, dass** das in der Zusammensetzung vorliegende Polymer ein Acrylsäure/Acrylamid-Copolymer mit 30 bis 100 % der Acrylsäurefunktionen in neutralisierter Form ist.

14. Verwendung nach einem der Ansprüche 1 bis 3 oder 5 bis 11 oder Zusammensetzung nach einem der Ansprüche 4 bis 11, **dadurch gekennzeichnet, dass** das Polymer ein Copolymer ist, das mindestens eine monomere Einheit umfasst, die eine oder mehrere starke Säurefunktionen trägt.

15. Verwendung oder Zusammensetzung nach Anspruch 14, **dadurch gekennzeichnet, dass** der molare Anteil der Monomereinheiten, die eine oder mehrere starke Säurefunktion(en) tragen, bezogen auf die Gesamtheit der Monomereinheiten, weniger als 50 % und vorzugsweise weniger als 30 % beträgt.

16. Verwendung oder Zusammensetzung nach Anspruch 14 oder 15, **dadurch gekennzeichnet, dass** die monomere(n) Einheit(en), die eine oder mehrere starke Säurefunktionen in freier Form tragen, ausgewählt ist (sind) aus Acrylamidoalkylsulfonsäuren wie 2-Acrylamido-2-methylpropansulfonsäure.

17. Verwendung oder Zusammensetzung nach Anspruch 14, 15 oder 16 **dadurch gekennzeichnet, dass** das in der Zusammensetzung vorliegende Polymer ein 2-Acrylamido-2-methylpropansulfonsäure/Sulfonsäure-Copolymer oder 2-Acrylamido-2-methylpropansulfonsäure/Acrylsäure/Acrylamid-Copolymer ist, wobei 30 bis 100 % der auf dem Polymer vorhandenen Säurefunktionen in neutralisierter Form vorliegen.

18. Verwendung nach einem der Ansprüche 1 bis 3 oder 5 bis 17 oder Zusammensetzung nach einem der Ansprüche 4 bis 17, **dadurch gekennzeichnet, dass** das Verzweigungsmittel ausgewählt ist aus Methylenbisacrylamid (MBA), Ethylenglykoldiacrylat, Polyethylenglykoldiacrylat, Diacrylamid, Cyanomethylacrylat, Vinyloxyethylacrylat, Vinyloxymethacrylat, Triallylamin, Formaldehyd, Glyoxal, Glycidylethern wie Ethylenglykol-Diglycidylether, Epoxid und ihrer Mischung.

19. Verwendung nach einem der Ansprüche 1 bis 3 oder 5 bis 18 oder Zusammensetzung nach einem der Ansprüche 4 bis 18, **dadurch gekennzeichnet, dass** die Menge des Verzweigungsmittels zwischen 5 und 10.000 ppm, bezogen auf die Gesamtmasse der Monomere, und vorzugsweise zwischen 100 und 5.000 ppm liegt.

20. Verwendung nach einem der Ansprüche 1 bis 3 oder 5 bis 19 oder Zusammensetzung nach einem der Ansprüche 4 bis 19, **dadurch gekennzeichnet, dass** die Polymerisationsreaktion in Gegenwart eines Wasser-in-Öl-Emulgators umgesetzt wird.

21. Verwendung nach einem der Ansprüche 1 bis 3 oder 5 bis 20 oder Zusammensetzung nach einem der Ansprüche 4 bis 20, **dadurch gekennzeichnet, dass** die Polymerisation mit einem Übertragungsmittel durchgeführt wird, ausgewählt aus Methanol, Isopropylalkohol, Natriumhypophosphit, 2-Mercaptoethanol, Natriummethansulfonat und ihrer Mischung.

22. Verwendung oder Zusammensetzung nach Anspruch 21, **dadurch gekennzeichnet, dass** die Menge des Übertragungsmittels zwischen 0 und 5.000 ppm, bezogen auf die Gesamtmasse der Monomeren, und vorzugsweise zwischen 10 und 2.500 ppm liegt.

23. Verwendung nach einem der Ansprüche 1 bis 3 oder 5 bis 22 oder Zusammensetzung nach einem der Ansprüche 4 bis 22, **dadurch gekennzeichnet, dass** auf die Polymerisation einer oder mehrere der folgenden Schritte folgt:

   - eine Verdünnung oder Konzentrierung der resultierenden Emulsion,
   - eine Isolierung, um das Polymer in Form eines Pulvers zu erhalten.

24. Verwendung nach einem der Ansprüche 1 bis 3 oder 5 bis 23 oder Zusammensetzung nach einem der Ansprüche 4 bis 23, **dadurch gekennzeichnet, dass** das in die Zusammensetzung eingebrachte verzweigte oder vernetzte Polymer eine Viskosität von 0,16 Gew.-% in entmineralisiertem Wasser aufweist, dessen pH-Wert mit Natriumhydroxid auf 7 +/-0,1 eingestellt ist, gemessen bei 25 °C mit einer Brookfield RVT-Vorrichtung (Drehzahl 20 U/min) in einem Bereich von 2.000 mPa.s bis 100.000 mPa.s, insbesondere einem Bereich von 3.000 mPa.s bis 50.000 mPa.s.

25. Verwendung nach einem der Ansprüche 1 bis 3 oder 5 bis 24 oder Zusammensetzung nach einem der Ansprüche 4 bis 24, **dadurch gekennzeichnet, dass** die Zusammensetzung zur topischen Anwendung geeignet ist.

26. Verwendung nach einem der Ansprüche 1 bis 3 oder 5 bis 25 oder Zusammensetzung nach einem der Ansprüche 4 bis 25, **dadurch gekennzeichnet, dass** die Zusammensetzung in Form einer Milch, Lotion, eines Gels, einer Creme, eines Cremegels, einer Seife, eines Schaumbades, eines Balsams, eines Shampoos oder einer Spülung vorliegt.

27. Verwendung nach einem der Ansprüche 1 bis 3 oder 5 bis 26 oder Zusammensetzung nach einem der Ansprüche 4 bis 26, **dadurch gekennzeichnet, dass** die Zusammensetzung einen Elektrolyten umfasst, vorzugsweise ausgewählt aus Pflanzenextrakten, die einwertige oder zweiwertige Ionen wie Fruchtsäuren, Wirkstoffe wie Hydroxysäuren wegen ihrer Anti-Aging-Wirkung, Feuchtigkeitsspender wie Pyrrolidoncarbonsäure, Chelatbildner wie Ethylendiamintetraessigsäure (EDTA), UV-Filter wie Phenylbenzimidazolsulfonsäure, bestimmte Konservierungsmittel oder Salze wie Alaunsalz enthalten.

**28.** Verwendung nach einem der Ansprüche 1 bis 3 oder 5 bis 27 oder Zusammensetzung nach einem der Ansprüche 4 bis 27, **dadurch gekennzeichnet, dass** die Zusammensetzung 0,01 Gew.-% bis 10 Gew.-% verzweigtes oder vernetztes Polymer, bezogen auf die Gesamtmasse der Zusammensetzung, und vorzugsweise 0,1 bis 5 Gew.-% verzweigtes oder vernetztes Polymer umfasst.

**29.** Verwendung nach einem der Ansprüche 1 bis 3 oder 5 bis 28 oder Zusammensetzung nach einem der Ansprüche 4 bis 28 **dadurch gekennzeichnet, dass** die Zusammensetzung mindestens einen Wirkstoff umfasst, ausgewählt aus Feuchtigkeitsspendern, Bräunungsmitteln, Sonnenschutzmitteln, Vitaminen, Spurenelementen, Anti-Falten- oder Anti-Aging-Mitteln, Pflanzenextrakten, Schlankheitsmitteln, Radikalfängern, Haarausfallmitteln, Anti-Schuppen-Mitteln, Reinigungstensiden, Hautpflegepolymeren, Weichmachern und pharmazeutischen Wirkstoffen wie Anti-Pilz-Mitteln, antibakteriellen Mitteln, entzündungshemmenden Mitteln, Muskelrelaxantien, Antibiotika, antiviralen Mitteln, Analgetika, Antihistaminika, juckreizhemmenden Mitteln, fiebersenkenden Mitteln, Anästhetika, Diagnostika, Hormonen, Hautwachstumsförderern, Pigmentmodulatoren, antiproliferativen Mitteln, antipsoriatischen Mitteln, Retinoiden, Anti-Akne-Mitteln, antineoplastischen Mitteln, phototherapeutischen Mitteln, keratolytischen Mitteln und ihren Analogen.

**30.** Verwendung nach einem der Ansprüche 1 bis 3 oder 5 bis 29 oder Zusammensetzung nach einem der Ansprüche 4 bis 29, **dadurch gekennzeichnet, dass** die Zusammensetzung mindestens ein Additiv und insbesondere mindestens ein Formulierungshilfsmittel umfasst, ausgewählt aus Chelatbildnern, PH-Neutralisierungsmitteln und -Einstellmitteln, Trübungsmitteln, Konservierungsmitteln, Spreizmitteln, Weichmachern, filmbildenden Polymeren, Antioxidantien, Duftstoffen, Reflexionsmitteln, Koaleszenzmitteln und Mischungen davon.

**31.** Verwendung nach einem der Ansprüche 1 bis 3 oder 5 bis 30 oder Zusammensetzung nach einem der Ansprüche 4 bis 30, **dadurch gekennzeichnet, dass** die Zusammensetzung eine Emulsion einer Ölphase in einer wässrigen Phase oder eine Emulsion einer wässrigen Phase in einer Ölphase ist.

**32.** Verwendung oder Zusammensetzung nach Anspruch 31, **dadurch gekennzeichnet, dass** die Ölphase aus einem pflanzlichen Öl oder Pflanzenöl, Silikonöl, fluoriertem Kohlenwasserstofföl, Kohlenwasserstofföl, Mineralöl, Polyisobuten, Isohexadecan, Capryl-/Caprintriglycerid, Cetaryloctanoat, $C_{12}$-$C_{14}$-Alkylbenzoat, oder einer von ihrer Mischung besteht.

**33.** Verwendung nach einem der Ansprüche 1 bis 3 oder 5 bis 32 oder Zusammensetzung nach einem der Ansprüche 4 bis 32, **dadurch gekennzeichnet, dass** die Zusammensetzung einen Wasser-in-Öl-Emulgator und/oder einen Öl-in-Wasser-Emulgator umfasst.

**34.** Verwendung nach einem der Ansprüche 1 bis 3 oder 5 bis 33 oder Zusammensetzung nach einem der Ansprüche 4 bis 33, **dadurch gekennzeichnet, dass** das verzweigte oder vernetzte Polymer wasserlöslich oder wasserquellbar ist.

**35.** Verwendung einer Zusammensetzung nach einem der Ansprüche 4 bis 34 zur kosmetischen Behandlung von Keratinmaterialien, wie Haut, Kopfhaut, Wimpern, Augenbrauen, Nägel, Haare und/oder Schleimhäute, mit Ausnahme jeglicher therapeutischer Behandlung.

**36.** Verwendung nach Anspruch 35, die das Auftragen der Zusammensetzung auf die Keratinmaterialien, und gegebenenfalls anschließendes Spülen mit Wasser umfasst.

**Claims**

**1.** The use, for producing a cosmetic or dermatological composition comprising at least one aqueous phase, of a branched or crosslinked polymer composed of the repetition of one or more monomeric units, with at least one of the monomeric units corresponding to a monomer comprising an acrylic group, and at least 30 mol% of the monomeric units bearing at least one weak acid function, possibly in neutralized form, said polymer being obtained:
by polymerization of an aqueous solution of one or more monomers in water-in-oil inverse emulsion, at least one of the monomers used being an acrylic monomer and one or more of the monomers used being a monomer bearing at least one weak acid function, the molar percentage of monomers bearing at least one weak acid function relative to all of the monomers used being at least 30%, the aqueous phase containing at least one monomer acting as branching agent, in such a manner that polymerization leads to a branched or crosslinked polymer, **characterized**

**in that**:

> i) the polymerization is carried out with a concentration of all the monomers in aqueous solution lying in the range 1.3 mmol to 3.6 mmol per gram of aqueous solution; and
> ii) during the polymerization, at most 20% of the acid functions present on the monomers having at least one acid function are in neutralized form.

**2.** The use, for thickening a cosmetic or dermatological composition comprising at least one aqueous phase, of a branched or crosslinked polymer composed of the repetition of one or more monomeric units, with at least one of the monomeric units corresponding to a monomer comprising an acrylic group, and at least 30 mol% of the monomeric units bearing at least one weak acid function, possibly in neutralized form, said polymer being obtained:
by polymerization of an aqueous solution of one or more monomers in water-in-oil inverse emulsion, at least one of the monomers used being an acrylic monomer and one or more of the monomers used being a monomer bearing at least one weak acid function, the molar percentage of monomers bearing at least one weak acid function relative to all of the monomers used being at least 30%, the aqueous phase containing at least one monomer acting as branching agent, in such a manner that polymerization leads to a branched or crosslinked polymer, **characterized in that**:

> i) the polymerization is carried out with a concentration of all the monomers in aqueous solution lying in the range 1.3 mmol to 3.6 mmol per gram of aqueous solution; and
> ii) during the polymerization, at most 20% of the acid functions present on the monomers having at least one acid function are in neutralized form.

**3.** The use according to claim 1 or claim 2, **characterized in that** the polymer includes a percentage of neutralized acid functions in the range 30% to 100% relative to all of the acid functions present on the polymer, which percentage is obtained by a step of at least partial neutralization of the acid functions present on the polymer and carried out after the polymerization, but before or after preparing the composition.

**4.** A cosmetic or dermatological composition comprising at least one aqueous phase and a branched or crosslinked polymer composed of the repetition of one or more monomeric units, with at least one of the monomeric units corresponding to a monomer comprising an acrylic group, and at least 30 mol% of the monomeric units bearing at least one weak acid function, at least partially in neutralized form, the percentage of neutralized acid functions relative to all the acid functions present on the polymer being lying in the range 30% to 100%, said polymer being obtained:

> by polymerization of an aqueous solution of one or more monomers in water-in-oil inverse emulsion, at least one of the monomers used being an acrylic monomer and one or more of the monomers used being a monomer bearing at least one weak acid function, the molar percentage of monomers bearing at least one weak acid function relative to all of the monomers used being at least 30%, the aqueous phase containing at least one monomer acting as branching agent, in such a manner that polymerization leads to a branched or crosslinked polymer, **characterized in that**:

>> i) the polymerization is carried out with a concentration of all the monomers in aqueous solution lying in the range 1.3 mmol to 3.6 mmol per gram of aqueous solution; and
>> ii) during the polymerization, at most 20% of the acid functions present on the monomers having at least one acid function are in neutralized form;

> the polymerization being followed by a step of at least partial neutralization of the acid functions present, carried out before or after incorporation of the polymer in the composition.

**5.** The use according to claim 1, 2 or 3, or a composition according to claim 4, **characterized in that**, during the polymerization, at most 10%, preferably at most 5%, and more preferably at most 2%, of the acid functions present on the monomers having at least one acid function are in neutralized form.

**6.** The use according to any one of claims 1 to 3 or 5, or a composition according to claim 4 or 5, **characterized in that** all of the acid functions present on the monomers are in free acid form during the polymerization.

**7.** The use according to any one of claims 1 to 3, 5 or 6, or a composition according to any one of claims 4 to 6, **characterized in that** the polymerization is carried out with a concentration of all the monomers in aqueous solution lying in the range 1.7 to 3.3 mmol per gram of aqueous solution.

8. The use according to any one of claims 1 to 3 or 5 to 7, or a composition according to any one of claims 4 to 7, **characterized in that** the polymer includes a molar percentage of monomeric units bearing one or more weak acid function(s), relative to all of the monomeric units bearing an acid function, of at least 50%, preferably of at least 70%, more preferably of at least 80%.

9. The use according to any one of claims 1 to 3 or 5 to 8, or a composition according to any one of claims 4 to 8, **characterized in that** all the monomers used for polymer preparation are monomers which have at least one ethylenically unsaturated bond.

10. The use according to any one of claims 1 to 3 or 5 to 9, or a composition according to any one of claims 4 to 9, **characterized in that** the monomeric unit(s) bearing at least one weak acid function, in free form, is/are chosen from acrylic acid, methacrylic acid, itaconic acid, crotonic acid, maleic acid, and fumaric acid, acrylic acid being preferred.

11. The use according to any one of claims 1 to 3 or 5 to 10, or a composition according to any one of claims 4 to 10, **characterized in that** the polymer is a copolymer including at least one neutral monomeric unit chosen from acrylamide, methacrylamide, N,N-dimethylacrylamide, N-vinylmethylacetamide, N-vinylformamide, vinyl acetate, diacetone acrylamide, N-isopropylacrylamide, N-[2-hydroxy-1,1-bis(hydroxymethyl)ethyl]propenamide, (2-hydroxyethyl) acrylate, (2,3-dihydroxypropyl) acrylate, methyl methacrylate, (2-hydroxyethyl) methacrylate, (2,3-dihydroxypropyl) methacrylate, and vinylpyrrolidone.

12. The use according to any one of claims 1 to 3 or 5 to 11, or a composition according to any one of claims 4 to 11, **characterized in that** all of the monomeric units bearing at least one acid function are monomeric units bearing one or more weak acid function(s).

13. The use or a composition according to claim 12, **characterized in that** the polymer present in the composition is an acrylic acid/acryamide copolymer with 30% to 100% acrylic acid functions in neutralized form.

14. The use according to any one of claims 1 to 3 or 5 to 11, or a composition according to any one of claims 4 to 11, **characterized in that** the polymer is a copolymer including at least one monomeric unit bearing one or more strong acid function(s).

15. The use or a composition according to claim 14, **characterized in that** the molar percentage in monomeric units bearing one or more strong acid function(s) relative to all of the monomeric units is less than 50%, and preferably less than 30%.

16. The use or a composition according to claim 14 or 15, **characterized in that** the monomeric unit(s) bearing one or more strong acid function(s), in free form, is/are chosen from acrylamidoalkylsulfonic acids such as 2-acrylamido-2-methylpropane sulfonic acid.

17. The use or a composition according to claim 14, 15, or 16, **characterized in that** the polymer present in the composition is a copolymer of 2-acrylamido-2-methylpropane sulfonic acid and acrylic acid or of 2-acrylamido-2-methylpropane sulfonic acid and acrylic acid and acrylamide, with 30% to 100% acid functions present on the polymer that are in neutralized form.

18. The use according to any one of claims 1 to 3 or 5 to 17, or a composition according to any one of claims 4 to 17, **characterized in that** the branching agent is chosen from methylenebisacrylamide (MBA), ethylene glycol diacrylate, polyethylene glycol dimethacrylate, diacrylamide, cyanomethyl acrylate, vinyloxyethyl acrylate, vinyloxy methacrylate, triallylamine, formaldehyde, glyoxal, glycidyl ethers such as ethylene glycol diglycidyl ether, and epoxies, and mixtures thereof.

19. The use according to any one of claims 1 to 3 or 5 to 18, or a composition according to any one of claims 4 to 18, **characterized in that** the amount of branching agent is between 5 ppm and 10000 ppm by weight, relative to the total weight of monomer, and preferably between 100 ppm and 5000 ppm.

**20.** The use according to any one of claims 1 to 3 or 5 to 19, or a composition according to any one of claims 4 to 19, **characterized in that** the polymerization reaction is carried out in the presence of a water-in-oil emulsifier.

**21.** The use according to any one of claims 1 to 3 or 5 to 20, or a composition according to any one of claims 4 to 20, **characterized in that** the polymerization is carried out with a transfer agent chosen from methanol, isopropyl alcohol, sodium hypophosphite, 2-mercaptoethanol, and sodium methallyl sulfonate, and mixtures thereof.

**22.** The use or a composition according to claim 21, **characterized in that** the amount of branching agent is between 0 ppm and 5000 ppm by weight, relative to the total weight of monomer, and preferably between 10 ppm and 2500 ppm.

**23.** The use according to any one of claims 1 to 3 or 5 to 22, or a composition according to any one of claims 4 to 22, **characterized in that** the polymerization is followed by one or more of the following steps:

- diluting or concentrating the resulting emulsion; and
- isolating to obtain the polymer in the form of a powder.

**24.** The use according to any one of claims 1 to 3 or 5 to 23, or a composition according to any one of claims 4 to 23, **characterized in that** when the branched or crosslinked polymer introduced into the composition is at 0.16% by weight in demineralized water at pH that was adjusted to 7 ±0.1 with sodium hydroxide, it presents viscosity as measured at 25°C with a Brookfield viscometer of the RVT type (rotation speed 20 t/min), lying in the range 2000 mPa.s to 100 000 mPa.s, in particular lying in the range 3000 mPa.s to 50 000 mPa.s.

**25.** The use according to any one of claims 1 to 3 or 5 to 24, or a composition according to any one of claims 4 to 24, **characterized in that** the polymerization is suitable for topical application.

**26.** The use according to any one of claims 1 to 3 or 5 to 25, or a composition according to any one of claims 4 to 25, **characterized in that** the composition is in the form of a milk, a lotion, a gel, a cream, a gel cream, a soap, a bubble bath, a balm, a shampoo, or a conditioner.

**27.** The use according to any one of claims 1 to 3 or 5 to 26, or a composition according to any one of claims 4 to 26, **characterized in that** the composition comprises an electrolyte, preferably selected from among vegetable extracts containing monovalent or divalent ions such as fruit acids, active ingredients such as hydroxy acids for their anti-ageing effect, moisturizing agents such as pyrrolidone carboxylic acid, chelating agents such as ethylenediaminetetraacetic acid (EDTA), UV filters such as phenylbenzimidazole sulfonic acid, certain preservatives, or also salts such as alum salts.

**28.** The use according to any one of claims 1 to 3 or 5 to 27, or a composition according to any one of claims 4 to 27, **characterized in that** the composition comprises in the range 0.01% to 10% by weight of branched or crosslinked polymer, relative to the total weight of the composition, and preferably in the range 0.1% to 5% by weight of branched or crosslinked polymer.

**29.** The use according to any one of claims 1 to 3 or 5 to 28, or a composition according to any one of claims 4 to 28, **characterized in that** the composition comprises at least one active agent chosen from moisturizing agents, tanning agents, sunscreens, vitamins, oligo-elements, anti-wrinkle or anti-ageing agents, botanical extracts, slimming agents, anti-radical agents, anti hair-loss agents, anti-dandruff agents, cleansing surfactants, skin-conditioning polymers, emollients and pharmaceutical active ingredients such as anti-fungal agents, antibacterial agents, anti-inflammatory agents, myorelaxants, antibiotics, antiviral agents, analgesics, antihistamines, antipruritic agents, antipyretic agents, anesthetic agents, diagnostic agents, hormones, skin growth enhancers, pigment modulators, antiproliferative agents, antipsoriatic agents, retinoids, anti-acne medicines, antineoplastic agents, phototherapeutic agents, keratolytic agents, and analogs thereof.

**30.** The use according to any one of claims 1 to 3 or 5 to 29, or a composition according to any one of claims 4 to 29, **characterized in that** the composition comprises at least one additive, and in particular at least one formulation aid, for example chosen from chelating agents, pH neutralization and adjustment agents, opacifiers, preservatives, leveling agents, emollients, film-forming polymers, antioxidants, perfumes, reflective agents, coalescing agents, and mixtures thereof.

**31.** The use according to any one of claims 1 to 3 or 5 to 30, or a composition according to any one of claims 4 to 30, **characterized in that** the composition is an emulsion of an oily phase in an aqueous phase or an emulsion of an aqueous phase in an oily phase.

32. The use or a composition according to claim 31, **characterized in that** the oily phase is made up of a vegetable or plant oil, a silicone oil, a fluorinated hydrocarbon oil, a hydrocarbon oil, a mineral oil, polyisobutene, isohexadecane, a caprylic/capric triglyceride, cetearyl octanoate, $C_{12}$-$C_{14}$ alkyl benzoate, or a mixture thereof.

33. The use according to any one of claims 1 to 3 or 5 to 32, or a composition according to any one of claims 4 to 32, **characterized in that** the composition comprises a water-in-oil emulsifier and/or an oil-in-water-emulsifier.

34. The use according to any one of claims 1 to 3 or 5 to 33, or a composition according to any one of claims 4 to 33, **characterized in that** the branched or crosslinked polymer is a water-soluble or water-swelling polymer.

35. The use of a composition according to claims 4 to 34 for cosmetic treatment of keratinous material such as the skin, the scalp, the eyelashes, the eyebrows, the nails, hair, and/or mucous membranes, excluding any therapeutic treatment.

36. The use according to claim 35, which use includes application of the composition to keratinous material, possibly followed by rinsing with water.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5004598 A
- WO 2005097834 A
- EP 0503853 A
- EP 1047716 A
- US 20030147825 A
- US 5216070 A
- US 5380465 A
- US 4539368 A
- US 4656222 A
- FR 2979821
- US 3755560 A
- US 4421769 A
- US 4704272 A
- US 4741855 A
- US 4788006 A
- US 5011681 A
- US 2658072 A
- US 2438091 A
- US 2528378 A
- EP 1710259 A
- EP 0603019 A
- WO 9221316 A
- DE 19523596
- EP 0715845 A
- EP 0604249 A
- EP 0576188 A
- WO 9307902 A
- WO 9221318 A
- WO 9427561 A
- WO 9809611 A

**Littérature non-brevet citée dans la description**

- *Chinese Chemical Letters,* 2002, vol. 13 (10), 993-996
- Harry's Cosmetology. Chemical Publishing Company Inc, 1982, 266
- Handbook of Cosmetic Science and Technology. Elsevier Science Publishing, 1993, 175
- **TOLD ; BYERS.** Volatile Silicone Fluids for Cosmetics. *Cosmetics and Toiletries,* Janvier 1976, vol. 91, 27-32
- **FRANCK S. ; D'AMELIO, SR.** Botanicais A Phytocosmetic Desck Reference. CRC Press LLC, 1999, 39
- **SEGARIN et al.** Cosmetics Science and Technology. 1890
- *Fed. Reg.,* 21 Mai 1999, vol. 64, 27666-27693
- Mc Cutcheon's detergents and Emulsifiers. Allured Publishing Corporation, 1996